(19) Europäisches Patentamt
European Patent Office
Office européen des brevets

(11) EP 1 476 162 B1

(12) **EUROPEAN PATENT SPECIFICATION**

(45) Date of publication and mention of the grant of the patent:
**18.04.2007 Bulletin 2007/16**

(21) Application number: **03702918.8**

(22) Date of filing: **17.02.2003**

(51) Int Cl.:
***A61K 31/496*** *(2006.01)* ***A61P 25/00*** *(2006.01)*

(86) International application number:
**PCT/IB2003/000642**

(87) International publication number:
**WO 2003/070246 (28.08.2003 Gazette 2003/35)**

(54) **CONTROLLED SYNTHESIS OF ZIPRASIDONE**

KONTROLLIERTE SYNTHESE VON ZIPRASIDON

SYNTHESE CONTROLEE DE ZIPRASIDONE

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **20.02.2002 US 358806 P**
**21.02.2002 US 359038 P**
**27.02.2002 US 360459 P**

(43) Date of publication of application:
**17.11.2004 Bulletin 2004/47**

(73) Proprietor: **Pfizer Products Inc.**
**Groton,**
**Connecticut 06340 (US)**

(72) Inventors:
- **BUSCH, Frank, Robert**
**Eastern Point Road,**
**Groton, CT 06340 (US)**
- **GROBIN, Adam, Worth**
**Chappel Hill, NC 27516 (US)**
- **HOWARD, Harry, Ralph, Jr.**
**Eastern Point Road,**
**Groton, CT 06340 (US)**
- **LEEMAN, Kyle, Robert**
**Eastern Point Road,**
**Groton, CT 06340 (US)**

(74) Representative: **Dekker, Henrike Cornelie Christine et al**
**Pfizer**
**European Pharma Patent Department**
**23-25, avenue du Docteur Lannelongue**
**75668 Paris Cedex 14 (FR)**

(56) References cited:
**EP-A- 0 584 903**
**EP-A- 0 586 191**
**EP-A- 0 931 547**
**WO-A-97/42191**
**US-A- 5 338 846**
**US-A- 5 359 068**
**US-A- 6 110 918**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Background of the Invention

Efforts are made to prepare pharmaceutical products of a high grade and with a minimum amount of impurities present. The control of impurities requires a study of various options to decide upon the reaction conditions and testing protocols necessary to insure that drugs which are administered to the public are pure.

Guidance given by regulatory bodies, including the United States Food and Drug Administration (FDA), suggests that impurities in drugs be identfied, if present, if they are at a level of 0.1% (that is, 1000 ppm) or greater for drug substances dosed at 2g/day or lower. (Note that ppm is parts per million, so that 1% =10,000 ppm; 0.1% = 1000 ppm; 0.01 % = 100 ppm; and 0.001 % = 10 ppm). For example, the FDA has indicated that identification of impurities below apparent levels of 0.1 % for a 2g/day-dosed drug substance is generally not considered necessary (Federal Register Vol. 65, No. 140 pp. 45085-45090, 45086 and45089 (July 20, 2000)). However, the FDA also points out that tighter controls may be necessary for some impurities, depending upon their specific properties (*Id*. at 45086). Furthermore, studies to obtain safety information for a proposed quantity of an impurity are recommended if the proposed quantity exceeds a qualification threshold of 0.05% (500 ppm for a drug substance dosed at 2g/day or lower (*Id*. at 45087 and 45089).

Ziprasidone (5-(2-(4-(1,2-benzisothiazol-3-yl-1-piperazinyl)-ethyl)-6-chloro-1,3-dihydro-2-(1H)-indol-2-one) is a potent antipsychotic agent and is useful for treating various disorders including schizophrenia, anxiety and migraine pain. Ziprasidone has been approved by the FDA for treatment of schizophrenia and goes by the brand name Geodon™ in the United States. Ziprasidone has also been indicated as useful for treating Tourette's Syndrome (United States Patent 6,127,373), glaucoma and ischemic retinopathy (EP 985414 A2), and psychiatric conditions including dementia of the Alzheimer's type, bipolar disorders, mood disorders, panic disorders, agoraphobia, social phobia, panic disorder, post-traumatic stress disorder, acute stress disorder, substance-induced anxiety disorder, anxiety disorders not otherwise specified, dyskinesias and behavioral manifestations of mental retardation, conduct disorder, and autistic disorder (United States Patent 6,245,766).

United States Patent 4,831,031 describes a genus of compounds encompassing ziprasidone and the synthesis of such compounds. Another method for synthesizing ziprasidone is described in United States Patent 5,206,366. A method for specifically synthesizing ziprasidone hydrochloride monohydrate is described in United States Patent 5,312,925. A method for synthesizing ziprasidone mesylate dihydrate is described in United States Patent 6,245,765; and a method for synthesizing ziprasidone mesylate trihydrate is described in United States Patent 6,110,918. United States Patents 5,338,846; 5,359,068; and 6,111,105 also describe methods for synthesizing ziprasidone and/or intermediates therefore.

The structure of ziprasidone can be depicted as:

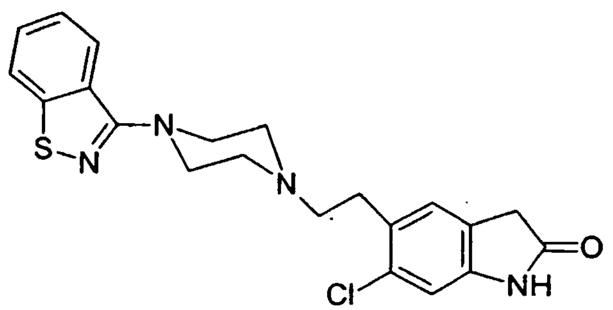

(H. Howard, et al., "Ziprasidone Hydrochloride", Drugs of the Future 1994, 19(6): 560-563. As can be seen from the structure above, the compound ziprasidone comprises a chlorine atom.

Methods of introducing halogens into organic compounds are summarized in many organic text books. For example, J. March, Advanced Organic Chemistry, 4th Edition, pp. 587-591, and references cited therein, has a discussion of halogenation chemistry. More specifically, formation of chloro-aromatic compounds are frequently formed by a variety of methods also well known to those skilled in the art, and again summarized in J. March, Advanced Organic Chemistry, 4th Edition, Chapter 11, "Aromatic Electrophilic Substitution". The chemistry to add a halogen, or more specifically a chlorine, to an aromatic group is thus well known to those skilled in the art. It is also known that such chemistry usually results in some mixtures of molecules, one of which is commonly the unreacted starting material not containing the chlorine atom. Further, over-chlorination is a problem well known to those skilled in the art; it is common to form some dichloro-compound impurities when the mono-chloro is desired and some trichloro-compound impurities when the dichloro- is desired. Over-chlorination is typically controlled by limiting the amount of the chlorinating reagent used. Unfortunately, control of over-chlorinated analogs in the drug substance by limiting the amount of chlorinating reagent utilized in the introduction of the aromatic chlorine substituent would be expected to result in more of a des-chloro impurity (unreacted starting material not containing the chlorine atom).

## Summary of the Invention

The des-chloro analog of ziprasidone is 5-[2-[4-(1,2)-benzisothiazol-3-yl)-1-piperazinyl]ethyl]-1,3-dihydro-2*H*-indol-2-one (hereinafter des-chloro ziprasidone). Based on the known methods for synthesizing halogenated aromatic compounds referred to above, any synthesized batch of ziprasidone drug substance will comprise some amount of a des-chloro ziprasidone impurity. Control of over-chlorinated analogs in the drug substance by limiting the amount of chlorinating reagent utilized in the introduction of the aromatic chlorine substituent would be expected to result in more of the des-chloro ziprasidone impurity.

The subject invention pertains to the techniques we have developed to control the synthesis of ziprasidone drug substance to insure that levels of des-chloro ziprasidone are at low levels. In our particular drug substance for use in pharmaceutical compositions, a level of not greater than about 100 ppm des-chloro ziprasidone consistently is met. However, our invention pertains to a method for obtaining ziprasidone compositions comprising levels of des-chloro ziprasidone of up to but not greater than about 1000 ppm and to methods for controlling the levels of des-chloro ziprasidone to up to but not greater than about 1000 ppm in a ziprasidone composition.

Thus the invention provides a method of synthesizing a ziprasidone composition that comprises an amount of des-chloro ziprasidone selected from not greater than about

A) 1000 ppm,
B) not greater than about 500 ppm, and
C) not greater than about 100 ppm

which method comprises:

a) obtaining one or more samples of one or more 6-chloro-1,3-dihydro-2*H*, indol-2-one batches;
b) measuring the level of oxindole impurity in each of the samples of (a);
c) selecting a 6-chloro-1,3-dihydro-2*H*-indol-2-one batch that comprises a level of oxindole of

    for (A), not greater than about 0.3% based on the measurement or measurements conducted in (b),
    for (B), not greater than about 0.15% based on the measurement or measurements conducted in (b), and
    for (C), not greater than about 0.03% based on the measurement or measurements conducted in (b); and

d) using the batch selected in (c) to synthesize said ziprasidone composition.

As used herein and unless otherwise indicated, the term "ziprasidone" includes ziprasidone free base and pharmaceutically acceptable salts of ziprasidone. A generic teaching of preparation of pharmaceutically acceptable salts of a genus of compounds including ziprasidone is provided in United States Patent 4,831,031 (see, for example, Column 3 thereof). In one embodiment, the ziprasidone in the composition of the present invention is ziprasidone free base. In another embodiment, the ziprasidone in the composition of the present invention is ziprasidone hydrochloride monohydrate. In another embodiment, the ziprasidone is ziprasidone mesylate dihydrate, and in another embodiment, the ziprasidone is ziprasidone mesylate trihydrate.

The term "ziprasidone drug substance", as used herein and unless otherwise indicated, refers to a ziprasidone composition, as defined above, that is used in the formulation of a pharmaceutical composition. Such pharmaceutical composition may contain pharmaceutical carriers, excipients, flavorings, and other ingredients that are known to be used in pharmaceutical compositions and as described in more detail below.

The pharmaceutical composition obtained by the method of the invention is for treating in a mammal, including a human, a disorder or condition selected from schizophrenia, anxiety, migraine pain, Tourette's Syndrome, glaucoma, ischemic retinopathy, dementia of the Alzheimer's type, a bipolar disorder, a mood disorder, agoraphobia, social phobia, panic disorder, post-traumatic stress disorder, acute stress disorder, substance-induced anxiety disorder, an anxiety disorders not otherwise specified (NOS), dyskinesias, a behavioral manifestation of mental retardation, conduct disorder, and autistic disorder comprising an amount of a ziprasidone drug substance which is a composition comprising ziprasidone and an amount of des-chloro-ziprasidone of not greater than about 1000 ppm, which amount of ziprasidone drug substance is effective in treating said disorder or condition, and a pharmaceutically acceptable carrier.

In one embodiment, the amount of des-chloro ziprasidone in the ziprasidone drug substance is not greater

than about 500 ppm. In a preferred embodiment, the amount of des-chloro ziprasidone in the ziprasidone drug substance is not greater about 100 ppm des-chloro ziprasidone.

In one embodiment, the amount of des-chloro ziprasidone in the ziprasidone drug substance is not greater than about 500 ppm. In a preferred embodiment, the amount of des-chloro ziprasidone in the ziprasidone drug substance is not greater about 100 ppm des-chloro ziprasidone,

This invention also provides a method of synthesizing a ziprasidone composition that comprises an amount of des-chloro ziprasidone of not greater than about 1000 ppm, which method comprises starting with a composition of a chlorinated reactant comprising a sufficiently low level of non-chlorinated impurity to synthesize said ziprasidone composition. In one embodiment, the chlorinated reactant is a composition of 6-chlorooxindole (6-chloro-1,3-dihydro-2*H*-indol-2-one).

Although there are many known routes to 6-chlorooxindole, starting materials therefore are typically a substituted 4-chlorotoluene or 1,4-dichloro-nitrobenzene (see, G. J. Quallich and P. M. Morrissey, Synthesis, 1993, 51-53; and references cited therein; and F. R. Busch and R. J. Shine, "Development of an Efficient Process to 6-Chlorooxindole", presented at the 208th ACS National Meeting in Washington D.C. in the Symposium on Technical Achievements in Organic Chemistry, 1994, (talk #126).). However, the concept of controlling chlorinated isomers, over-chlorination, or des-chloro impurities for the synthesis of 6-chlorooxindole is not described in the prior art. Other methods of synthesizing 6-chlorooxindole can be determined by a person of ordinary skill in the art, and such methods are included in the step of obtaining a batch of 6-chlorooxindole for the above-described method of this invention. Furthermore, a batch of 6-chlorooxindole can be obtained by purchase from manufacturers of organic chemicals, for example Plaistow, Ltd., Little Island, County Cork, Ireland or Finorga, Route de Givors, 38670 Chasse-Sur-Rhone, France.

As used herein, a "6-chlorooxindole batch" is a composition consisting essentially of 6-chlorooxindole, which composition may contain low levels of impurities, one of which may be oxindole.

The level of oxindole impurity in a sample of a batch of 6-chlorooxindole can be determined using standard analytical techniques known to those of ordinary skill in the art. For example, the level of oxindole impurity may be determined by normal phase HPLC, reverse phase HPLC, or gas chromatography methods.

A specific method for determining the level of oxindole in a sample of a 6-chlorooxindole batch is referred to herein as "Detection Method B" and is provided in the "Detailed Description of the Invention" Section of this application below.

This invention also provides a method of synthesizing a ziprasidone composition that comprises an amount of des-chloro ziprasidone of not greater than about 1000 ppm, which method comprises:

a) acylating a composition comprising 6-chloro-1,3-dihydro-2*H*-indol-2-one and an oxindole impurity with chloroacetyl chloride by Friedel-Crafts Acylation to synthesize a composition comprising 6-chloro-5-(chloroacetyl)-1,3-dihydro-2H-indol-2-one;
b) treating the composition resulting from (a) to reduce of the oxo of the chloroacetyl group therein to form a composition comprising 6-chloro-5-(2-chloroethyl)-1,3-dihydro-2*H*-indol-2-one and a 5-(2-chloroethyl)-1,3-dihydro-2*H*-indol-2-one impurity;
c) isolating a sample of the composition resulting from (b);
d) measuring the quantity of 5-(2-chloroethyl)-1,3-dihydro-2*H*-indol-2-one impurity in the isolated sample from (c);
e) determining whether or not the quantity in (d) is not greater than about 0.28%; and
f) purifying by recrystallization and/or reslurry the composition resulting from (b) if the quantity measured in (d) is greater than about 0.28% until the quantity of the 5-(2-chloroethyl)-1,3-dihydro-2H-indol-2-one impurity is not greater than about 0.28%, and synthesizing a ziprasidone composition from the composition so purified; or,
g) if the quantity in (d) is not greater than about 0.28%, synthesizing a ziprasidone composition from the composition of (b).

In a preferred embodiment, the composition of ziprasidone prepared according to the method in the preceding paragraph comprises an amount of des-chloro ziprasidone of not greater than about 500 ppm, with the value of 0.28% provided in steps (e) and (f) being adjusted accordingly to about 0.14%. In a more preferred embodiment, the composition of ziprasidone prepared according to the method in the preceding paragraph comprises an amount of des-chloro ziprasidone of not greater than 100 ppm, with the value of 0.28% in steps (e) and (f) being adjusted accordingly to about 0.028%.

Measurement of 5-(2-chloroethyl)-1,3-dihydro-2*H*-indol-2-one, as in step (d) in the method described in the preceding paragraphs, can be conducted by standard analytical chemistry techniques, for example reverse phase HPLC or other suitable chromatographic methods.

However, in a preferred embodiment, the 5-(2-chloroethyl)-1,3-dihydro-2*H*-indol-2-one is measured in step (b) by Detection Method A, described below.

In one embodiment, the purification of a composition comprising 6-chloro-5-(2-chloroethyl)-1,3-dihydro-2*H*-indol-2-one in step (f) is by reslurry. Reslurry is a process similar to recrystallization, but where all of the material is not

completely dissolved. The composition comprising 6-chloro-5-(2-chloroethyl)-1,3-dihydro-2*H*-indol-2-one in step (f) may however be purified by recrystallization, reslurry, or a combination thereof. A preferred method of purification of 6-chloro-5-(2-chloroethyl)-1,3-dihydro-2*H*-indol-2-one is by recrystallization and/or reslurry in an aqueous miscible solvent, preferably acetonitrile/water.

This invention also provides a HPLC method, called "Detection Method A" herein, for measuring the quantity of 5-(2-chloroethyl)-1,3-dihydro-2*H*-indol-2-one in a composition comprising 6-chloro-5-(2-chloroethyl)-1,3-dihydro-2*H*-indol-2-one.

More specifically, this invention provides a method, "Detection Method A", using HPLC for measuring the quantity of 5-(2-chloroethyl)-1,3-dihydro-2*H*-indol-2-one in a composition comprising 6-chloro-5-(2-chloroethyl)-1,3-dihydro-2*H*-indol-2-one, which method comprises

a) preparing sample solution from said composition comprising 6-chloro-5-(2-chloroethyl)-1,3-dihydro-2*H*-indol-2-one by dissolving a portion of said composition in an organic solvent, followed by dilution with an organic solvent of the dissolved portion so that a concentration (weight/volume), based on the weight of said portion and volume of solvent, of about 1 mg/mL is obtained:
b) running the sample solution through a HPLC column comprising a stationary phase consisting essentially of a cyano bonded phase using a mobile phase consisting essentially of (75:13-17:8-12 v/v/v/) 0.05 M $KH_2PO_4$. pH=from 5.5-6.5, Acetonitrile:Methanol; at a column temperature of from 30˚ C to 40˚ C; with detection by UV light at 254nm UV:
c) detecting a peak appearing at from between 8 to 10 minutes on a chromatogram resulting from (b);
d) measuring the peak area (named $A_c$) of the peak detected in (c);
e) preparing a standard from a composition consisting essentially of 5-(2-chloroethyl)-1,3-dihydro-2*H*-indol-2-one by dissolving and diluting a portion of said composition in an organic solvent such that the concentration (weight/volume) of 5-(2-chloroethyl)-1,3-dihydro-2*H*-indol-2-one, based on the weight of the portion and volume of the solvent, is about equal to a selected fraction value at or above which detection of 5-(2-chloroethyl)-1,3-dihydro-2*H*-indol-2-one in the composition comprising 6-chloro-5-(2-chloroethyl)-1,3-dihydro-2*H*-indol-2-one is desired;
f) running the standard through a stable-bond cyano HPLC column using a mobile phase consisting essentially of (75:13-17:8-12 v/v/v/) 0.05 M $KH_2PO_4$, pH=from 5.5-6.5,:Acetonitrile:Methanol; at a column temperature of from 30˚ C to 40˚ C; with detection by UV light at 254nm UV;
g) measuring the peak area (named $A_{pur1}$) of the peak on a chromatogram resulting from (f); and
h) calculating the quantity of 5-(2-chloroethyl)-1,3-dihydro-2*H*-indol-2-one in the composition comprising 6-chloro-5-(2-chloroethyl)-1,3-dihydro-2*H*-indol-2-one by

i) calculating the Response Factor for 5-(2-chloroethyl)-1,3-dihyrdo-2*H*-indol-2-one according to the following formula:

$$R_{pur1}=(A_{pur1})(DF)/(W_{pur1})(PF)$$

wherein : $A_{pur1}$ is as defined above;
$W_{pur1}$ = weight of composition in the standard;
PF = potency factor for 5-(2-chloroethyl)-1,3-dihyrdo-2*H*-indol-2-one; and
DF = dilution factor for the standard; and
ii) calculating the %w/w of 5-(2-chloroethyl)-1,3-dihyrdo-2*H*-indol-2-one according to the following formula:

$$\%w/w=(A_c)(DF)(100)/(R_{pur1})(W_{S2})$$

wherein : $A_c$ is as defined above;
$R_{pur1}$ = Response Factor calculated in (h)(i) above;
$W_{S2}$ = weight of the portion of the composition used in step (a); and
DF = dilution factor for sample solution.

Organic solvents that are useful in steps (a) and (e) include, but are not limited to THF (tetrahydrofuran), methanol, acetonitrile, or the mobile phase described in step (a). Other organic solvents may also be useful in this method. In a preferred embodiment of the HPLC method described in the preceding paragraph, the sample solution is

dissolved in THF and is subsequently diluted using the mobile phase. In another preferred embodiment, the standard is prepared by diluting the composition consisting essentially of 5-(2-chloroethyl)-1,3-dihydro-2*H*-indol-2-one in THF.

In another preferred embodiment, a flow rate of about 1.0mL/min is used in the HPLC method. In another preferred embodiment, injection volumes of the standard and of the sample solution of at least about 20 μL, more preferably about 20 μL, are used.

In another preferred embodiment of the above-described HPLC method, the column temperature is 35° C. In another preferred embodiment, the ratio of $KH_2PO_4$:acetonitril:methanol is 75:15:10. In another preferred embodiment, the pH of the $KH_2PO_4$ is 6.0.

A "stable-bond cyano column", as used herein, means a HPLC column comprising a stationary phase consisting essentially of a cyano bonded phase. Stable-bond cyano columns are known to those of ordinary skill in the art. Such HPLC columns are readily available to those of ordinary skill in the art from commercial sources, for example the HPLC column Zorbax™ (Mac-Mod Analytical, P.O. Box 2600, 127 Commons Court, Pennsylvania 19317, USA).

The "Potency Factor" used in the calculation in step (h) in the above-described method refers to the purity of the composition consisting essentially of 5-(2-chloroethyl)-1,3-dihydro-2*H*-indol-2-one with respect to the 5-(2-chloroethyl)-1,3-dihyrdo-2*H*-indol-2-one therein. The Potency Factor can be determined by a person of ordinary skill in the art by deducting quantities, if any, of substances for which detection is typically conducted by a person of ordinary skill when analyzing the purity of an organic composition. Such substances include, for example, water, solvent or solvents, and "residue on ignition" (i.e. inorganic matter, for example sodium or potassium). Detection and quantification of such substance can be determined by a person of ordinary skill in the art. Hence, taking into account such substances, a Potency Factor for a composition consisting essentially of 5-(2-chloroethyl)-1,3-dihydro-2*H*-indol-2-one may be, for example, 98% 5-(2-chloroethylyl.3-dihyrdo-2*H*-indol-2-one.

The "Dilution Factors" (for the sample solution and for the standard) used in the calculation in step (h) in the above-described method refer to the amount by which the composition being analyzed and the composition consisting essentially of 5-(2-chloroethyl)-1,3-dihyrdo-2*H*-indol-2-one was diluted in steps (a) and (e), respectively. Hence, the Dilution Factor for the sample solution will be the volume of solvent used to prepare the sample solution in step (a) of the method. For example, if 80 mg of a composition comprising 6-chloro-5-(2-chloroethyl)-1,3-dihydro-2*H*-indol-2-one is used in step (a), and 80 mL of solvent accordingly, then the Dilution Factor will be 80. The Dilution Factor for Standard A will depend on the value selected in step (e). For example, if detection of 5-(2-chloroethyl)-1,3-dihyrdo-2*H*-indol-2-one at or above about 100 ppm is selected, then the concentration of the 5-(2-chloroethyl)-1,3-dihydro-2*H*-indol-2-one in the standard will be about 0.0001. If, for example, 20 mg of the composition consisting essentially of 5-(2-chloroethyl)-1,3-dihydro-2*H*-indol-2-one is used in step (e), then the Dilution Factor for the standard is 20/.0001, or $2x10^5$. As another example, if detection of 5-(2-chloroethyl)-1,3-dihydro-2*H*-indol-2-one at or above about 500 ppm is selected, then the concentration of the 5-(2-chloroethyl)-1,3-dihydro-2*H*-indol-2-one in the standard will be about 0.0005. If, for example, 20 mg of the composition consisting essentially of 5-(2-chloroethyl)-1,3-dihydro-2*H*-indol-2-one is used in step (e), then the Dilution Factor for the standard will be 20/.0005, or $4x10^4$. As another example, if detection of 5-(2-chloroethyl)-1,3-dihydro-2*H*-indol-2-one at or above about 1000 ppm is selected, then the concentration of the 5-(2-chloroethyl)-1,3-dihydro-2*H*-indol-2-one in the standard will be about 0.001. If, for example, 20 mg of the composition consisting essentially of 5-(2-chloroethyl)-1,3-dihydro-2*H*-indol-2-one is again used in step (e), then the Dilution Factor for the standard will be 20/.001, and the Dilution Factor for the standard will be $2x10^4$.

This invention also provides a method of synthesizing a ziprasidone composition that comprises an amount of des-chloro ziprasidone of not greater than about 1000 ppm, which method comprises:

a) reducing a composition comprising 6-chloro-5-(chloroacetyl)-1,3-dihydro-2*H*-indol-2-one and a 5-(chloroacetyl)-1,3-dihydro-2*H*-indol-2-one impurity by treatment with triethylsilane in the presence of a strong acid to obtain a composition comprising 6-chloro-5-(2-chloroetheyl-1,3-dihydro-2*H*-indol-2-one and a 5-(2-chloroetheyl-1,3-dihydro-2*H*-indol-2-one impurity; and

b) synthesizing a composition comprising ziprasidone from the composition resulting from (a). In a preferred embodiment, the strong acid in step (a) comprises trifluoroacetic acid or methanesulfonic acid.

In another preferred embodiment, the method further comprises

i) isolating prior to step (b) a sample of the composition from (a), and measuring the quantity of 5-(2-chloroethyl)-1,3-dihydro-2*H*-indol-2-one impurity in said sample;

ii) determining whether or not the quantity in (i) is not greater than about 0.28%; and

iii) purifying by recrystallization and/or reslurry the composition from (a) if the quantity in (i) is greater than about 0.28% until the quantity of the 5-(2-chloroethyl)-1,3-dihydro-2*H*-indol-2-one impurity is not greater than about 0.28%, and then proceeding to step (b) using the composition from (a) so purified; or

iv) if the quantity in (i) is not greater than about 0.28%, then proceeding to step (b).

In another embodiment, the method is for synthesizing a ziprasidone composition comprising not greater than 500ppm of des-chloro ziprasidone. The value of 0.28% used for analyzing the sample from step (a) may be adjusted accordingly to 0.14%. In another embodiment, the aforementioned method is for synthesizing a ziprasidone composition comprising not greater than 100ppm. The value of 0.28% used for analyzing the method may likewise be adjusted accordingly to 280 ppm.

In another embodiment of this method, the quantity of 5-(2-chloroethyl)-1,3-dihydro-2*H*-indol-2-one in the sample of the composition comprising 6-chloro-5-(2-chloroethyl)-1,3-dihydro-2*H*-indol-2-one is measured by a method comprising Detection Method A.

The purification of the composition comprising 6-chloro-5-(2-chloroetheyl-1,3-dihydro-2*H*-indol-2-one in step (a)(ii) of the above method is by reslurry and/or recrystallization. The recrystallization and/or reslurry are in a suitable solvent mixture, preferably an aqueous miscible solvent, and more preferably a mixture of acetonitrile/water.

This invention also provides a method of synthesizing a ziprasidone composition that comprises an amount of des-chloro ziprasidone of not greater than about 1000 ppm, which method comprises:

a) purifying a composition comprising 6-chloro-1,3-dihydro-2*H*-indol-2-one and an oxindole impurity until a composition comprising not greater than about 0.3% of said oxindole impurity is obtained; and
b) using the composition resulting from (a) to synthesize a ziprasidone composition.

In one embodiment, the composition in (a) is purified until a composition comprising not greater than about 0.15% of oxindole impurity is obtained, and a composition that comprises an amount of des-chloro ziprasidone of not greater than about 500 ppm is synthesized.

In another embodiment, the composition in (a) is purified until a composition comprising not greater than about 0.03% of oxindole impurity is obtained, and a composition that comprises an amount of des-chloro ziprasidone of not greater than about 100 ppm is synthesized.

Methods of purification of the composition comprising -chloro-1,3-dihydro-2*H*-indol-2-one and an oxindole impurity that can be used in this invention include extraction and/or recrystallization and/or reslurry. In one embodiment the purification is by recrystallization and/or reslurry from organic solvents. See, for example, G. J. Quallich and P. M. Morrissey, *supra,* and references cited therein; and F. R. Busch and R. J. Shine, *supra.*

This invention also provides a method of synthesizing a ziprasidone composition that comprises an amount of des-chloro ziprasidone of not greater than about 1000 ppm, which method comprises:

a) recrystallizing and/or reslurrying a composition comprising 6-chloro-5-(2-chloroethyl)-1,3-dihydro-2*H*-indol-2-one and a 5-(2-chloroethylyl-1,3-dihydro-2*H*-indol-2-one impurity until a composition comprising not greater than about 0.3% of said 5-(2-chloroethyl)-1,3-dihydro-2*H*-indol-2-one impurity is obtained; and
b) using the composition resulting from (a) to synthesize a ziprasidone composition.

In one embodiment, the composition in (a) is recrystallized and/or reslurried until a composition comprising not greater than about 0.15% of said 5-(2-chloroethyl)-1,3-dihydro-2*H*-indol-2-one impurity is obtained, and a composition that comprises an amount of des-chloro ziprasidone of not greater than about 500 ppm is synthesized.

In another embodiment, the composition in (a) is recrystallized and/or reslurried until a composition comprising not greater than about 0.03% of 5-(2-chloroethyl)-1,3-dihydro-2*H*-indol-2-one impurity is obtained, and a composition that comprises an amount of des-chloro ziprasidone of not greater than about 100 ppm is synthesized.

In a preferred embodiment, the recrystallization/reslurrying conditions in step (a) of this method comprise use of polar organic solvents and/or polar organic solvents mixed with a small volume of water. Preferably, the recrystallization and/or reslurry is with an aqueous miscible solvent, for example acetonitrile/water.

The terms "treatment", "treating", and the like, refers to reversing, alleviating, or inhibiting the progress of the disorder or condition to which such term applies, or one or more symptoms of such disorder or condition. As used herein, these terms also encompass, depending on the condition of the patient, preventing the onset of a disorder or condition, or of symptoms associated with a disorder or condition, including reducing the severity of a disorder or condition or symptoms associated therewith prior to affliction with said disorder or condition. Thus, "treatment", as used herein, can refer to administration of a compound of the invention to a subject that is not at the time of administration afflicted with the disorder or condition. "Treating" thus also encompasses preventing the recurrence of a disorder or condition or of symptoms associated therewith.

"Mammal", as used herein, and unless otherwise indicated, means any mammal. The term "mammal" includes, for example and without limitation, dogs, cats, and humans.

The term "about", when used herein in, for example, "less than 'about' 1000 ppm" means within a range of plus or minus 10% of the value to which the term is being applied.

Detailed Description of the Invention

One method for synthesizing ziprasidone, as noted above, is taught in United States Patent 5,206,366, which has been incorporated herein by reference. This Patent teaches that ziprasidone can be synthesized according to the method depicted in Scheme 1, below. Scheme 2, below, illustrates the mechanism by which des-chloro ziprasidone forms from any oxindole impurity in the starting reactant if a synthesis according to Scheme 1 is conducted. "IPO" in the following Schemes indicates isopropyl alcohol:

**Scheme 1**

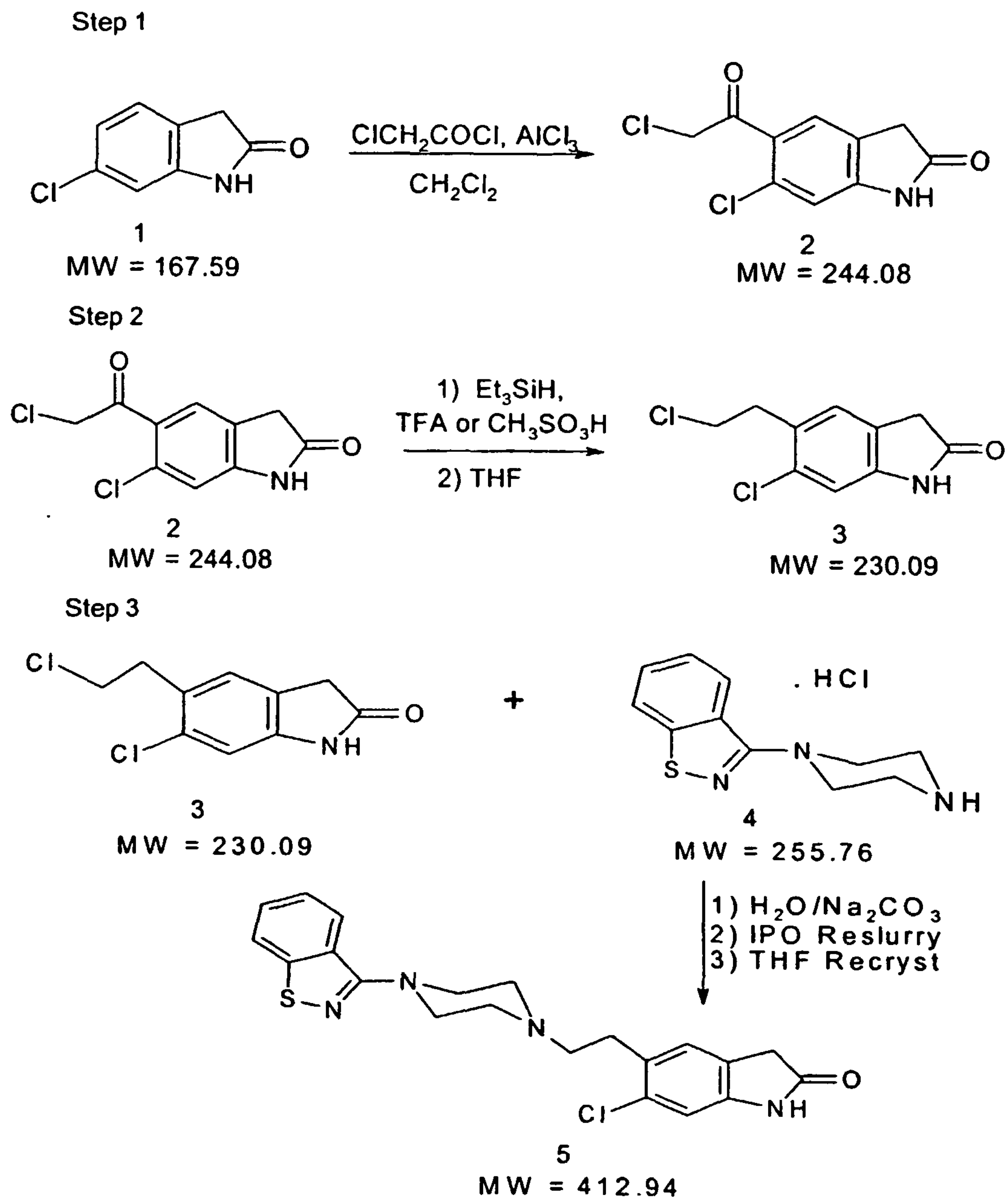


1
MW = 167.59

2
MW = 244.08

2
MW = 244.08

3
MW = 230.09

3
MW = 230.09

4
MW = 255.76

5
MW = 412.94

Step 4

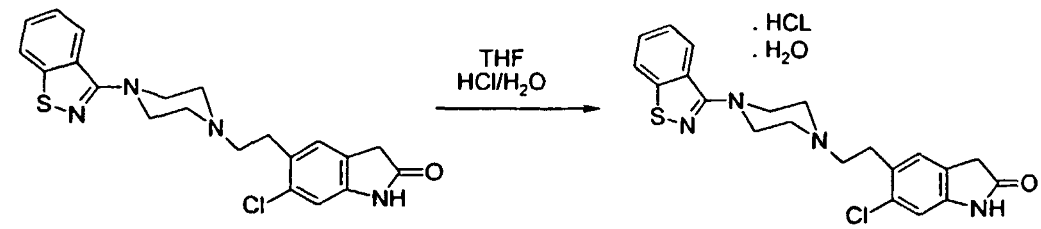


5
MW = 412.94

ziprasidone hydrochloride monohydrate
MW = 467.42

**Scheme 2**

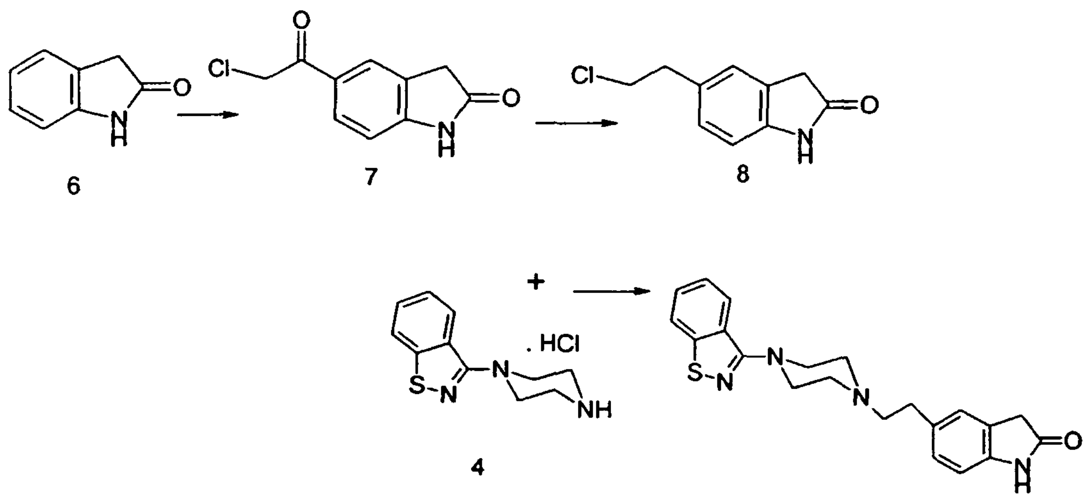

6 7 8

4 9

One control strategy we identified was determining the purging of the des-chloro impurities during chemical synthesis of ziprasidone, and then setting sufficient limits on the quality of the starting reactant used to synthesize ziprasidone. During a synthesis of ziprasidone, one or more of the intermediates undergoes extraction and crystallization during the reaction work-ups. Although each intermediate and it's corresponding des-chloro impurity are structurally similar and have similar solubility, there are slight differences in the physio-chemical properties. Thus, we conducted purging experiments to determine the amount of des-chloro impurity that is removed by the extraction and recrystallization processing operations.

Referring to Schemes 1 and 2, Compound **6,** oxindole, a potential impurity in a batch of the starting material, Compound 1, 6-chlorooxindole, may proceed under reaction conditions to result in compound **9,** des-chloro ziprasidone. Surprisingly, we found a greater than three-fold purge of Compound **6** and its subsequent analogs during the reaction and isolation conditions of the synthetic process. Therefore, commercial supplies of Compound 1 containing up to 0.03% of Compound **6** will result in ziprasidone drug substance containing less than 0.01% (100 ppm) of Compound **9.**

The control of a maximum limit of about 0.03% (300 ppm) Compound **6** in the purchased Compound **1** is justified based on the levels of Compound **6** detected in previous lots of Compound **1,** purging information obtained during the development of the synthesis, and measurements obtained during the processing of these intermediates.

As noted above, oxindole can be detected by standard analytical techniques. One specific method we have

found for determining oxindole in a composition comprising 6-chlorooxindole is as follows and will be referred to herein as "Detection Method B":

DETECTION METHOD B:

PRINCIPLE:

Normal phase liquid chromatography (LC) is used to separate **Compound 1** from its potential impurities. Comparison of the peak areas and retention times for samples and the working standard of **Compound 1** provides a quantitative assay and identification test for **Compound 1.** Comparison of the peak areas of the specified impurities, if present, against dilute solutions of the impurities, provides a quantitative measure of their abundance.

APPARATUS:

1. Standard laboratory equipment.
2. Suitable liquid chromatograph

   a. Pump - constant flow delivery
   b. UV detector - 254 nm
   c. Injector capable of making 50 μL injections
   d. Data acquisition system

3. Column:

   Waters Associates Nova-Pak silica column, 4 micron particles, 150 x 3.9 mm (i.d.).
   (2 columns placed in series or equivalent length of 300 mm), or equivalent

4. Balance capable of weighing 50 mg ± 0.1 mg - e.g. Mettler AE240

REAGENTS:

1. Hexane - HPLC grade
2. Tetrahydrofuran (THF)
3. Isopropanol (IPO) - HPLC grade
4. 15-crown-5 (1,4,7,10,13-pentaoxacyclopentadecane)
5. Working standard of Compound 6 (oxindole)

CHROMATOGRAPHIC CONDITIONS:

1. Flow Rate: 1.5 mL/min
2. Injection Volume: 50 μL
3. Detection Wavelength: 254 nm
4. Mobile Phase: hexane / isopropanol / tetrahydrofuran / 15-crown-5 1000/9/9/0.5 (v/v/v/v)
5. Column Temperature: Ambient (about 23°C)

Under the above cited conditions, **Compound 6** will elute between 15-19 minutes. The elution time for **Compound 1** is between 17-21 minutes.

MOBILE PHASE PREPARATION:

In a 2 liter flask, add in the following order: 1000 mL of hexane, 9 mL of isopropanol, 9 mL of tetrahydrofuran, and 0.5 mL of 15-crown-5. Mix well and degas under vacuum with sonication or stirring for about 20 seconds.

SAMPLE AND STANDARDS PREPARATION:

1. **Compound 1** Sample and Working Standard Preparation:

Weigh (in duplicate), to the nearest 0.1 mg, about 20 mg of **Compound 1** working standard and samples and add to individual 100 mL flasks. Duplicate weights should be prepared for the working standard and each sample lot. Pipet 10 mL of THF to each flask, sonicate for about 1 min, add enough mobile phase to fill each flask approximately 80 percent of capacity, shake, and allow to equilibrate to room temperature. Dilute to volume (QS) with mobile phase. (See operator's note #1). Designate the working standard solution as **POT1**. Designate the sample solution as **A1**.

2. Preparation of Working Standard for **Compound 6**

| COMPOUND | CODE | SAMPLE WEIGHT | FLASK |
|---|---|---|---|
| **Compound 6** | Ox | 10 mg | 100mL |

Pipet 10 mL of THF to the flask, sonicate for 1 minute, add enough mobile phase to fill the flask to approximately 80 percent of capacity, shake, and allow to warm to room temperature. Dilute to volume with additional mobile phase and label as **PUR 1.**

B. Further dilute the **PUR1** solution to yield **PUR2** solutions as follows:

Label the flask with as solution **PUR2**.

| COMPOUND | CODE | TRANSFER VOLUME | FLASK SIZE |
|---|---|---|---|
| **Compound 6** | Ox | 2 mL **PUR1** | 100 mL |

Dilute the solution (**PUR2**) flask to volume with mobile phase.

C. Prepare the final concentration of the oxindole impurity by diluting the **PUR2** solution to yield a **PUR3** solution:

| COMPOUND | CODE | TRANSFER VOLUME | FLASK SIZE |
|---|---|---|---|
| **Compound 6** | Ox | 2 mL **PUR2** | 100mL |

Pipet the indicated volume of **PUR2** solution into the flask and add 10 mL of THF. Fill each flask to approximately 80 percent of capacity with mobile phase, and allow to warm to room temperature. Dilute to volume with mobile phase. Label the flask as solution **PUR3**.

SYSTEM SUITABILITY:

A complete system suitability should be determined prior to the initial assay and after any significant change to the system. For these criteria, see the SYSTEM SUITABILITY section that follows in this procedure.

SYSTEM SUITABILITY PRIOR TO EACH ANALYSIS:

The following criteria must be achieved prior to every analysis made with this procedure.

1. Calculate the resolution between **Compound 1** and **Compound 6** by using the preparation specified in the SYSTEM SUITABILITY section.
2. Verify that an adequate limit of quantitation (LOQ) is achieved. Using the solution **PUR3**, perform 2 replicate injections. The areas of the **Compound 6** peaks should agree within 25%. Calculate the % area agreement as follows:

$$\text{\% area agreement} = \frac{2(A-B)X100}{A+B}$$

Where: A = Area of oxindole (**Compound 6**) peak in injection 1 B = Area of oxindole (**Compound 6**) peak in injection 2
3. For the working standard solution of **Compound 1 POT1** measure the retention time for **Compound 1**. The retention time should be within the range of 17-21 minutes.

PROCEDURE:

1. Inject purity standard **PUR3,** four samples **(A), PUR3,** four samples, etc. No more than four samples may be injected between standards. For the purity standard **(PUR3)**, measure the peak area and retention time of each specified impurity. For each sample injection, measure the retention time and the chromatographic peak area of each peak observed (See operator's note #3).

IDENTITY TEST:

This test is satisfactorily met if the **Compound 1** sample solution **(A)** under test exhibits a major peak whose retention time is identical (± 2%) to that of the **Compound 1** working standard solution **(POT1).**

CALCULATIONS:

PURITY:

1. For each sample, establish the presence of oxindole, if any, according to the relative retention time indicated in the table found in the CHROMATOGRAPHIC CONDITIONS section, and by comparison to the retention time of the peaks in the respective purity standard **(PUR3).** Their identities are established if the retention time does not differ by more than 2% (sample peak versus peak in the specified impurity standard). Specified impurities are to be quantified with the purity standard **(PUR3).**
2. Calculate the standard response factor for oxindole:

$$SR_i = (A_i)(DF)/(W_i)(PF_i)$$

where:

$SR_i$ = Standard response factor of oxindole
$A_i$ = Area of the specified impurity in PUR3
$w_i$ = Weight (mg) of the oxindole
$PF_i$ = Potency Factor of the oxindoleworking standard (e.g., 0.993).
DF = Dilution factors for oxindole:
= 250,000

3. Calculate the percent of oxindole in the following manner:

$$\%\ oxindole = (A(s))(DF)(100)/(SR_i\ (avg))(W_s)$$

where:

$SR_i$ (avg) = Average standard response for the oxindole working standard.
$W_s$ = Weight of the oxindole sample in mg
A(s) = Area of the oxindole in the sample.
DF = Dilution factor = 100
100 = Conversion to %

System Suitability:

The criteria set forth below establish chromatographic conditions that ensure the system is operating in a manner suitable to carry out the procedure. If any of these are failed, appropriate adjustments to the system should be made prior to proceeding. System suitability should be assessed prior to the first analysis on the system or after any significant change (e.g., replacement of column, repair of autosampler, etc.).

1. Reproducibility

Perform five replicate injections of the **Compound 1** working standard solution. Measure the peak area for **Compound 1.** The relative standard deviation (coefficient of variation) of the peak areas for **Compound 1** should not exceed 2.0%.
Perform six replicate injections of the purity standard solution **(PUR3)** containing **Compound 6.** Measure the peak area for **Compound 6.** The relative standard deviation (coefficient of variation) of the peak areas for **Compound 6** should not exceed 15%.

2. Efficiency

Calculate the number of theoretical plates (N) for the chromatographic column using one representative injection of the **Compound 1** working standard solution. The number of theoretical plates should be equal to or greater than 6,000.

$$N = 16\,(t/W)^2$$

3. Peak Asymmetry

Calculate the peak asymmetry (T) for the **Compound 1** peak using one representative injection of the working standard solution. The peak asymmetry should not be more than 2.0.

$$T = (W_{0.05} / 2f)$$

4. Resolution

Calculate the resolution (R) between **Compound 1** and **Compound 6** by preparing a sample of **Compound 1** spiked with 0.1% (w/w) of **Compound 6** as follows:
Prepare a solution of **Compound 1** as directed above under Step #1 of the SAMPLE AND STANDARDS PREPARATION. Before diluting to volume, add 10 mL of the **PUR2** solution prepared in Step **B** in the preparation of impurities standards section above.
The resolution between **Compound 1** and **Compound 6** should be > 1.0.

$$R = (2\,(t_2 - t_1))/(W_2 + W_1)$$

5. The definitions of the terms used in this section are:

t = Retention time measured from time of injection to time of elution of peak maximum
W = Width of peak measured by extrapolating the relatively straight sides to baseline

OPERATOR NOTES:

1. The time required to equilibrate normal phase columns generally is longer than for reversed phase columns. A new column initially should be washed with 4 liters of mobile phase. The last peak pair to be resolved during equilibration are **Compound** 6 and **Compound 1.** Make several injections of the reference for equilibration.
2. Reference and Sample make-up (diluting and warming to room temperature) should be done at the same time to insure that volumes are the same.
3. A THF blank (10 mL THF to volume with mobile phase) may be run to insure that there is no interference with any peaks of interest.

As described above, this invention also provides an additional method to control for low des-chloro ziprasidone by ensuring low des-chloro Step 2 intermediate (Compound **8** of Scheme 2, 5-(2-chloroethyl)-oxindole). This is an important control point, due to the opportunity to monitor the process following the reduction of the carbonyl. Reductive conditions also result in hydro-dehalogenation, and thus in synthesis depicted in Scheme 1 loss of the chlorine desired at the C-6 position.

Analytical methodology, the HPLC method described above, was developed to evaluate 6-chloro-5-(2-chloroethyl)-1,3-dihydro-2*H*-indol-2-one, compound 3 of Scheme 1, for assay and purity. One important function of this methodology, is to detect ppm levels of compound **8**; the des-chloro impurity, in the isolated material. The procedure "Detection Method A" set forth below exemplifies how an analyst might specifically apply this HPLC detection method to quantitate low levels of compound **8** without interference from other process-related impurities. This Example is not intended to and should not be construed to limit the invention described more fully herein and claimed below:

**Detection Method A:**

Following system suitability, the following analytical chromatographic HPLC method can be used to quantify the levels of des-chloro (Compound **8)** in Compound **3.**

APPARATUS:

1.Suitable HPLC, equipped with standard equipment
2.Column heater, capable of operating at 35 ˚C *e.g.*, BAS Temperature controller Model LC22A
3.Mobile phase preheater block: *e.g.*, Bioanalytical Systems, Inc. (BAS), Cat # EW8146
Note: This is required to improve column efficiency.
4. Column - Zorbax-SB-CN (Catalog No. 883975.905) 15 cm length x 4.6 mm I.D. (Available from Mac Mod Analytical, Chadds Ford, PA.)

REAGENTS:

1. 0.05 **M** Monobasic Potassium Phosphate ($KH_2PO_4$), pH = 6.0 Buffer Solution
Dissolve 6.8 g $KH_2PO_4$ in one liter of purified water. Adjust the pH of solution to 6.0 ± 0.1 with 5 **N** potassium hydroxide solution. Larger volumes may be prepared as needed.
2. Mobile Phase: (75 : 15 : 10 v/v/v) 0.05 **M** $KH_2PO_4$, pH= 6.0 : Acetonitrile:Methanol
Filter and degas under reduced pressure with stirring or ultrasonic agitation for about 5 minutes. Larger volumes of mobile phase may be prepared using the appropriate amounts of the components.

CHROMATOGRAPHIC CONDITIONS:

| Parameter | - | Set Point | Variation |
|---|---|---|---|
| Mobile Phase | - | as above | ± 2% ACN and MeOH |
| Column Temperature | - | 35 ˚C | ±5 ˚C |
| Detection | - | UV, 254nm | ± 5% |
| Flow Rate | - | 1.0 mUmin | ±0.1 mL/min |
| Injection Volume | | 20 µL | permissible |

(continued)

| Parameter | - | Set Point | Variation |
|---|---|---|---|
| Quantification Method | - | Area | - |
| Run Time | - | 60 minutes | Approximately |

Under the above-cited conditions Compound 8 will elute in approximately 8-10 minutes. Relative retention times of specified impurities are tabulated below.

| Compound | Rr |
|---|---|
| Compound **1** | 0.36 |
| Compound **2** | 0.45 |
| **Compound 8** | 0.49 |
| Compound **3** | 1.00 |

**Note:** Relative Retention Time (Rr) = retention time of specified impurity peak relative to retention time of Compound **3**

PREPARATION OF REFERENCE STANDARD SOLUTIONS:

Compound 8 Standard: Compound 8 standard into a 200 mL volumetric flask. Add approximately 20 mL THF and sonicate until sample has completely dissolved (approx. 1 min.). Dilute to volume with methanol. Mix well with additional shaking and inversions. Identify this as Compound 8 solution **F1.** The concentration of Compound 8 in solution **F1** is about 0.1 mg/mL.

Dilute 2 mL of **F1** to 100 mL with methanol and mix well. Identify this as Compound **8** solution **F2**.

The concentration of Compound **8** in solution **F2** is about 0.002 mg/mL.

PREPARATION OF SAMPLE SOLUTIONS:

Compound 3 Sample (for Compound **8** determination):

Prepare one test solution per sample. Weigh approximately 50 mg (record to the nearest 0.1 mg) of the Compound **3** sample into a 50 mL volumetric flask. Add approximately 20 mL of THF and sonicate until the sample has completely dissolved (approx. 2 min.). Dilute to volume with mobile phase. Mix well with additional shaking and inversions. Identify this as Compound **3,** Solution I (Sample Solution I). The concentration of solution I (Sample Solution I) is about 1.0 mg/mL.
Note: Solution I is stable for up to 24 hours under normal laboratory conditions.

SYSTEM SUITABILITY:

A complete system suitability should be determined prior to the initial assay and after any significant change to the system. For these criteria, see the SYSTEM SUITABILITY section at the end of this procedure.

SYSTEM SUITABILITY CHECK PRIOR TO EACH ANALYSIS:

The following criteria must be achieved prior to every analysis made with this procedure.

1. Calculate the resolution (R) between COMPOUND 2 and COMPOUND 8 using the **PUR1** standard. The resolution between this pair of peaks should be ≥ 1.0.

2. Verify that an adequate limit of quantitation (LOQ) is achieved. Using solution **PUR1,** perform 2 replicate injections. The COMPOUND 8 peak areas should agree within ± 20%. Calculate the % area agreement as follows:

$$\%\ \text{area agreement} = \frac{2(A-B) \times 100}{A+B}$$

A = COMPOUND 8 peak area from the 1 st injection.
B = COMPOUND 8 peak area from the 2nd injection.

3. For the standard solution of COMPOUND 3 **A1**, measure the retention time for COMPOUND 3. The retention time should be within the range of 16 - 24 minutes.

PROCEDURE:

Determine HPLC system suitability using the guidelines and procedures for running system suitability presented later in this test procedure. LOQ, resolution and retention time tests, as described in the SYSTEM SUITABILITY CHECK PRIOR TO EACH ANALYSIS sections, must be performed each time the system is used. The remaining criteria for system suitability should be assessed prior to the first analysis on the system and after any significant change

EVALUATION OF COMPOUND 8 CONTENT IN COMPOUND 3 SAMPLE:

(Solution (I))

Inject 20 μL aliquots of the sample solution **(I)**. Measure the area of the chromatographic peak of Compound **8** from each injection. Determine the Compound **8** levels present in the test sample **(I)** as described in the CALCULATIONS section.

CALCULATIONS:

Calculation of COMPOUND 8 Content:

1.Determine the response factor for COMPOUND 8 as follows:

$$R_{pur1} = \frac{A_{pur1} \times DF}{W_{pur1} \times PF}$$

where:

$A_{pur1}$ = Peak area of the impurity (COMPOUND 8) in PUR1
$W_{pur1}$ = Weight of the impurity (COMPOUND 8) in PUR1
PF = Potency factor for COMPOUND 8 or COMPOUND 2 (*e.g.*, 0.993)
DF = Dilution Factor - COMPOUND 8⇒ $2 \times 10^5$

2. Determine COMPOUND 8 content as follows:

$$\%\ w/w = \frac{A_c \times 50 \times 100}{R_{pur1} \times W_{S2}}$$

where:

$A_c$ = Peak area of COMPOUND 8 in Sample I

$R_{pur1}$ = Response factor for COMPOUND 8 in standard *
$W_{s2}$ = Weight of COMPOUND 3 in sample I in mg
50 = Dilution Factor
100 = Conversion to percent

* Use the average response factor for all **PUR1** injections made throughout the analysis.

SYSTEM SUITABILITY:

The criteria set forth below establish chromatographic conditions that ensure the system is operating in a manner suitable to carry out the procedure. If any of these are failed, appropriate adjustments to the system should be made prior to proceeding. System suitability should be assessed prior to the first analysis on the system or analysis on the system or after any significant change (*e.g.*, replacement of column, repair of autosampler, etc.).

1. Precision of Injection

Assay: Perform 5 replicate injections of the COMPOUND 3 standard **A1.** Measure the area of each COMPOUND 3 peak. The relative standard deviation of the peak areas should not exceed 1.0%.
Purity Evaluation: Perform 6 replicate injections of the **PUR1** standard solution. Measure the area of each COMPOUND 8 peak. The relative standard deviation of the peak area should not exceed 10%.

2. Efficiency

Calculate the number of theoretical plates (N) for the chromatographic column using the COMPOUND 3 peak in standard **A1.** The number of theoretical plates should not be less than 5000 when calculated by the tangent method.

$$N = 16\left(\frac{t}{W}\right)^2$$

t = Retention time measured from time of injection to time of elution of peak maximum.
W = Width of peak measured by extrapolating the relatively straight sides to baseline.

3. Retention Time

Measure the retention time for the COMPOUND 3 peak in standard **A1.** The retention time for the COMPOUND 3 peak should be within the range of 16 - 24 minutes.

4. Peak Asymmetry

Calculate the peak asymmetry (T) for the COMPOUND 3 peak in standard **A1**. The peak asymmetry should not be more than 2.0.

$$T = \frac{W_{0.05}}{2f}$$

T = Tailing factor
$W_{0.05}$ = Peak width at 5% height
f = Distance from the peak maximum to the leading edge of the peak, measured at 5% peak height

5. Resolution

Calculate the resolution (R) between COMPOUND 2 and COMPOUND 8 in **PUR1.** The resolution between this pair of peaks should be ≥ 1.0.

$$R = \frac{2(t_2 - t_1)}{W_1 + W_2}$$

t = Peak retention time
W = Peak width at baseline (measured by extrapolation to the baseline of tangents to the inflection points, for each of component)

As part of the development of this chemistry, multiple alternative synthetic methods were analyzed for the reduction of compound **2** to compound **3** in Scheme 1 above. For example, catalytic hydrogenation of the carbonyl of Compound **2** was tested using 5% palladium on carbon, palladium on alumina, platinum on carbon, or platinum on alumina. The palladium experiments were repeated at 10% catalyst loading, and in each case dehalogenation was a problem. We found that triethylsilane (TES) in the presence of a strong acid gives reduction of the carbonyl, without contaminate hydro de-halogenation. Therefore, this type of reduction is preferred for the production of compound **3.** Further, triflouroacetic acid or methanesulfonic acid have been found to be preferable strong acids for this chemistry, due to avoiding the formation of des-chloro impurities such as Compound **8.**

A purification was developed to re-process lots which we considered high in the des-chloro analogs. Processes were developed to rework Compounds **1** or **3.** We designed and conducted an experimental program testing purification of the starting material, each intermediate and the final drug to determine the most efficient point in the synthesis of ziprasidone to remove des-chloro compounds if present. We found that it is most efficient to remove the impurities by recrystallization and/or reslurry of Compounds **1** or **3.** Recrystallization of Compounds **2, 4,** or the final drug were very inefficient, giving very small reductions in the levels of the des-chloro impurities.

Specifically, many conditions were tested to purify Compound **3** including, acetonitrile, methylene chloride/toluene, ethyl acetate/hexanes, isopropyl alcohol, toluene, THF, isopropyl alcohol/DMAC (dimethylacetamide), methanol, isopropyl alcohol/acetic acid, and acetonitrile/water. The purification experiments to ascertain the preferred recrystallization and reslurry conditions for Compound 3 are summarized in Table 1, below. Overall most of the solvents tested were inefficient at removing Compound **6** from Compound **3.** The recrystallization/reslurry from acetonitrile/water was superior to the other procedures tested, giving a reduction in the level of the impurity from ~1300 ppm to ~300 ppm. Thus this recrystallization and/or reslurry is preferred for control of the des-chloro impurity 5-(2-chloroethyl)-1,3-dihydro-2*H*-indol-2-one.

**Table 1: Purification of Compound 3**

| Conditions | Yield | Color | Compound 8 (ppm) |
|---|---|---|---|
| Acetonitrile 10v | 92.0% | Off-white | 870 |
| $CH_2Cl_2$/Toluene 5:5 | 94.5% | Off-white | 1200 |
| EtOAc Distill add Hexane | 98.0% | White | 1100 |
| Isopropyl alcohol 20 vol | 89.3% | Off-white | 770 |
| Toluene 20 vol | 96.7% | Off-white | 930 |
| $CH_3CN/H_2O$ 9:1, ¾ hr | 92.7% | Off-white | 500 |
| $CH_3CN$ 112 vol, 5% Darco | 90.5% | Off-white | 430 |
| THF, Darco KB-B | 76.6% | White | 780 |
| THF | 81.2% | White | 720 |
| $CH_3CN/H_2O$ 9:1, 4 hrs | 94.5% | White | 440 |
| IPO/DMAC 7:3 | 78.5% | White | 480 |
| 10 vol $CH_3OH$ | 94.8% | White | 840 |
| IPO/HOAc 4:2 | 92.5% | Pink | 540 |
| $CH_3CN/H_2O$ 8:2, 18 hrs | 95.8% | White | 240 |
| $CH_3CN/H_2O$ 9:1, 18 hrs | 94.3% | White | 230 |

The ziprasidone drug substance of this invention may be administered as a neuroleptic agent as indicated herein as described in, for example, United States Patent 4,831,031, *supra.* Administration to a mammalian subject, including a human, may be alone or, preferably, in combination with pharmaceutically acceptable carriers or diluents in a pharmaceutical composition, in accordance with standard pharmaceutical practice. The pharmaceutical compositions may be administered orally or parenterally including intravenously or intramuscularly. Suitable pharmaceutical carriers include solid diluents or fillers, and sterile aqueous solutions and various organic solvents. The pharmaceutical compositions are then readily administered in a variety of dosage forms, such as tablets, powders, lozenges, syrups, and injectable solutions. These pharmaceutical compositions, if desired, may contain additional ingredients such as flavorings, binders and excipients. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid materials of a similar type may also be employed as fillers in soft and hard filled gelatin capsules. Preferred materials for this include lactose or milk sugar and high molecular weight polyethylene glycols. When aqueous suspensions or elixirs are desired for oral administration, the ziprasidone drug substance therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solution or suspension of the ziprasidone drug substance in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

The effective dosage of ziprasidone depends on the intended route of administration and other factors such as the indication being treated and the age and weight of the subject, as generally known. In general, a daily dosage will be in the range of from about 0.5mg of ziprasidone drug substance to about 500 mg, in single or divided doses, preferably from about 10 mg to about 200 mg per day. Presently, Geodon™ is approved in the United States for schizophrenia treatment in capsule form for oral administration comprising the ziprasidone hydrochloride monohydrate form of ziprasidone. The capsules are available in 20, 40, 60, and 80 mg of ziprasidone drug substance dosage forms. A typical daily dose for schizophrenia treatment based on a weight of about 70kg for a patient is preferably from about 20 mg twice per day to about 100 mg ziprasidone druge substance twice per day, more preferably about 20 mg twice per day to about 80 mg twice per day. However, it is appreciated that the dose and dosing regimen of ziprasidone drug substance may be varied from the aforementioned ranges and regimens by a physician of ordinary skill in the art, depending on the particular circumstances of any specific patient.

The following Examples illustrate the present invention. It is to be understood, however, that the invention, as fully described herein and as recited in the claims, is not intended to be limited by the details of the following Examples.

## EXAMPLES

### Example 1: Synthesis of Ziprasidone

#### Step 1: Friedel-Crafts Acylation of 6-chloro-1,3-dihydro-2*H*-indol-2-one

Methylene chloride (310 L) and aluminum chloride (172.3 kg) were combined. Chloroacetyl chloride (66.7 kg) was added, and the resulting mixture was stirred for 45 minutes. 6-Chloro-1,3-dihydro-2*H*-indol-2-one (61.8 kg) was added. The reaction mixture was stirred at 28 to 32° C for 19.5 hours and then cooled to 15 to 20 C. Water (805 L) was cooled to 5 to 10 C. The reaction was quenched by the slow addition of the reaction mixture to the cold water. After the quench was complete, the mixture was heated to reflux, and the methylene chloride was removed by atmospheric distillation at 43 to 57° C. The resulting mixture was cooled to 15 to 20° C and stirred for 1 hour. The solids were isolated by filtration and washed with water (114 L) followed by methanol (114 L).- The solids were dried in a suitable dryer.

6-Chloro-5-(chloroacetyl)-1,3-dihydro-2*H*-indol-2-one, yield: 91.3 kg (101.4%). Note: A weight yield in excess of 100% resulted due to small amounts of residual salts which were removed in the following step.

The resulting 6-chloro-5-(chloroacetyl)-1,3-dihydro-2*H*-indol-2-one was carried through the following step in portions, one of which is detailed below.

#### Step 2: Trifluoroacetic Acid/Silane Reduction of 6-Chloro-5-(chloroacetyl)-1,3-dihydro-2*H*-indol-2-one

Trifluoroacetic acid (278 kg) and (74.2 kg) were combined and stirred slowly at 24 to 28° C. Triethylsilane (77.9 kg) was charged to the stirring mixture. The reaction temperature was allowed to exotherm slightly during this addition

and was maintained between 50 to 62° C during the reaction period. , The reaction mixture was stirred for 8 hours, cooled to 38 C, and sampled for reaction completion. The reaction mixture was stirred at 50 to 54° C for an additional 3 hours. After the reaction was determined to be complete, the reaction mixture was cooled to 18° C, and quenched with water (594 L). The resulting slurry was stirred for 30 minutes at 10 to 15° C, and the solids were isolated by filtration. The product was rinsed from the tank and the product cake was washed with water (83 L) followed by methanol (76 L).

In each of two batches of equal size, tetrahydrofuran (742 L). Darco KB-B (1.9 kg), and the wet product cake were combined and heated to reflux. The resulting mixture was stirred at reflux for 30 minutes and filtered through a sparkler filter (precoated with filteraid) at 50 to 60° C to remove the carbon. The tank and sparkler were rinsed with hot tetrahydrofuran (38 L). Following the filtration the two batches were combined. The solution was concentrated *in vacuo* and stirred at 4 to 5° C for 1 hour. The solids were isolated by filtration and washed with cold tetrahydrofuran (38 L). The solids were dried *in vacuo* at 45 to 73° C until a loss on drying of 0.45% was achieved, giving 6-Chloro-5-(2-chloroethyl)-1,3-dihydro-2*H*-indol-2-one, yield: 60.1 kg (85.9%).

The resulting 6-chloro-5-(2-chloroethyl)-1,3-dihydro-2*H*-indol-2-one was combined with material of comparable quality and carried through the following step.

<u>Step 3: Coupling of 6-Chloro-5-(2-chloroethyl)-1,3-dihydro-2*H*-indol-2-one and 3-(1-Piperazinyl)-1,2-benzisothiazole Monohydrochloride</u>

Water (780 L) and sodium carbonate (126.0 kg) were combined and the mixture was stirred to dissolve 3-(1-Piperazinyl)-1,2-benzisothiazole monohydrochloride (155.0 kg) and 6-chloro-5-(2-chloroethyl)-1,3-dihydro-2*H*-indol-2-one (150.4 kg) were added, and the reaction mixture was heated to reflux (~100° C). After 24 and 28 hours, the reaction slurry was sampled for reaction completion assay. The reaction was determined to be complete after the assay of the second sample. Water (1251 L) was added and the slurry was cooled to temperatures between 18 to 22° C. The solids were isolated by filtration and washed with water (302 L). The water wet solids were combined with isopropanol (940 L) and the resulting mixture was stirred for approximately 2 hours at ambient temperature. The solids were isolated by filtration, washed with isopropanol (89 L), and dried *in vacuo* at less than 43° C, giving 5-[2-[4-(2,3-Benzisothiazol-3-yl)-1-piperazinyl]ethyl]-6-chloro-1,3-dihydro-2*H*-indol-2-one, yield: 202.8 kg (80.8%).

The resulting 5-[2-[4-(2,3-benzisothiazol-3-yl)-1-piperazinyl]ethyl]-6-chloro-1,3-dihydro-2*H*-indol-2-one was divided into two portions. These batches were carried separately through the following additional purification and resulted in material of comparable quality. The processing of one of these batches is detailed below.

<u>Step 3R: Purification of 5-[2-[4-(2,3-Benzisothiazol-3-yl)-1-piperazinyl]ethyl]-6-chloro-1,3-dihydro-2*H*-indol-2-one</u>

5-[2-[4-(2,3-Benzisothiazol-3-yl)-1-piperazinyl]ethyl]-6-chloro-1,3-dihydro-2*H*-indol-2-one (51 kg), filteraid (4 kg) and tetrahydrofuran (2678 L) were combined. The mixture was heated to reflux (~65° C) for ~1 hour, filtered while maintaining the temperature above 55° C, and rinsed with tetrahydrofuran (570 L).. The product rich filtrate was partially concentrated *in vacuo.* 5-[2-[4-(2.3-Benzisothiazo)-3-yl)-1-piperazinyl]ethyl]-6-chloro-1,3-dihydro-2*H*-indol-2-one (51 kg), filteraid (4 kg) and tetrahydrofuran (2675 L) were combined. The mixture was heated to reflux (~65° C) for ~1 hour, filtered while maintaining the temperature above 55° C, and rinsed with tetrahydrofuran (560 L). The product rich filtrate was combined with the partially concentrated mixture above and concentrated *in vacuo.* The resulting mixture was cooled to 0 to 5° C. The solids were isolated by filtration, washed with filtered tetrahydrofuran (113 L), and dried *in vacuo* at less than 41° C, giving ziprasidone, free base, yield: 79.3 kg (77.7 %).

A portion of the batch was combined with material of comparable quality which had been recrystallized separately and the batch was carried through the following step.

**<u>Example 2: Crystallization Salt Formation of Ziprasidone Hydrochloride Monohydrate</u>**

Tetrahydrofuran (2715 L), water (307 L), and 5-[2-[4-(2,3-benzisothiazol-3-yl)-1-piperazinyl]ethyl]-6-chloro-1,3-dihydro-2*H*-indol-2-one (100.0 kg) were combined, heated to reflux (~ 64° C), and stirred for -30 minutes. The solution was filtered and rinsed with tetrahydrofuran (358 L).

Water (203 L) and concentrated hydrochloric acid (29 L) were combined and stirred at ambient temperature. The resulting aqueous hydrochloric acid solution was charged to the 5-[2-[4-(2,3-benzisothiazol)-3-yl)-1-piperazinyl] ethyl]-6-chloro-1,3-dihydro-2*H*-indol-2-one solution over a period of 27 minutes. The reaction mixture was cooled to temperatures between 1 and 5° C over a period of ~2 hours. The mixture was stirred between 1 and 5 C for ~10 hours. The solids were isolated by filtration, washed with cold tetrahydrofuran (358. L), and dried until a water content of 4.1% was obtained.

Ziprasidone Hydrochloride Monohydrate, yield: 108.6 kg (96.0 % weight yield)

The solids were milled on a Bauermeister mill.

**Example 3: Purification of 6-Chloro-5-(2-chloroethyl)-1,3-dihydro-2H-indol-2-one To Remove 5-(2-Chloroethyl)-1,3-dihydro-2H-indol-2-one**

A 100 mL round bottom flask equipped with a magnet stirrer and reflux condenser was charged with 4.0 g (17.4 mmoles) of -6-chloro-5-(2-chloroethyl)-1,3-dihydro-2H-indol-2-one (Compound 3) and 36 mL of acetonitrile and 4.0 mL of water were added. The slurry was gently heated and stirred overnight (~18 hrs at ~78˚ C). The heating was then removed and the slurry cooled to 0 to 5˚ C, and stirred for an additional hour. The product was collected by filtration, washed with a small portion of acetonitrile and the product dried under vacuum at 50˚ C, to give 3.77 g (94.3% yield) of 6-chloro-5-(2-chloroethyl)-1,3-dihydro-2H-indol-2-one. The level of the des-chloro impurity had been reduced from 1280 ppm to 230 ppm.

**Example 4: Experimental Determination of Purge Factor for Compound 6 (1,3-Dihydro-2*H*-indol-2-one)**

A batch of 6-chloro-1,3-dihydro-2*H*-indol-2-one which contained a very high content of 1,3-dihydro-2*H*-indol-2-one was selected. This was intentionally selected so that higher levels of the impurity would be easier to measure, and to determine the purge factor for this impurity. An additional reason for this strategy of starting with material which was very high in the impurity for purposes of determining the purge factor of the impurity was to avoid having the material purge to less than the limit of analytical detection during the synthesis; thus resulting in a zero value in the final product. Since the purge factor is a ratio, it is not meaningful to divide by a zero result. (The material with the high level of impurity was used for this experiment but was NOT subsequently used in any studies with human subjects.) A batch of 6-chloro-1,3-dihydro-2*H*-indol-2-one which contained 4000 ppm of 1,3-dihydro-2*H*-indol-2-one was processed through the standard synthetic process according to Examples 1 and 2 above.

Following the first two steps of the synthesis, the level of the corresponding des-chloro impurity was measured, using the method described. It was found that 1700 ppm of 5-(2-chloroethyl)-1,3-dihydro-2*H*-indol-2-one (Compound **8** of Scheme 2, above) was present in 6-chloro-5-(2-chloroethyl)-1,3-dihydro-2*H*-indol-2-one (Compound **3** of Scheme 1, above). The processing was continued to 5-[2-[4-(1,2)-benzisothiazol-3-yl)-1-piperazinyl]ethyl]-6-chloro-1,3-dihydro-2*H*-indol-2-one hydrochloride monohydrate, where it was determined that 600 ppm of 5-[2-[4-(1,2)-benzisothiazol-3-yl)-1-piperazinyl]ethyl]-1,3-dihydro-2*H*-indol-2-one (Compound **9** of Scheme 2, above) was present.

Thus the purge factor through the entire synthesis for the des-chloro analogs was from 4000 ppm to 600 ppm, or approximately a 6-fold decrease. Minor run to run variations in processing can lead to small differences in the yield and quality of the materials produced. A 20% error in the reproducibility of the impurity formation, that is if 500 ppm in one run expecting between 400 and 600 ppm in other experiments, is then allowed for. In the case of the synthesis described in Examples 1 and 2, with 5 processing steps, the additive experimental error could result in as much as a 2-fold difference in the level of the impurity. Thus, for the purpose of setting the upper limit, where the drug is going to be used by human subjects a conservative 3-fold purge factor was utilized. Therefore, to insure that the product produced would not contain over 100 ppm of 5-[2-[4-(1,2)-benzisothiazol-3-yl)-1-piperazinyl]ethyl]-1,3-dihydro-2*H*-indol-2-one (Compound **9**), a limit of 300 ppm of 1,3-dihydro-2H-indol-2-one (Compound **6**) in 6-chloro-1,3-dihydro-2H-indol-2-one (Compound **1**) was determined.

**Claims**

**1.** A method of synthesizing a ziprasidone composition that comprises an amount of des-chloro ziprasidone selected from not greater than about

A) 1000 ppm.
B) not greater than about 500 ppm, and
C) not greater than about 100 ppm

which method comprises:

a) obtaining one or more samples of one or more 6-chloro-1,3-dihydro-*2H*-indol-2-one batches;
b) measuring the level of oxindole impurity in each of the samples of (a);
c) selecting a 6-chloro-1,3-dihydro-2*H*-indol-2-one batch that comprises a level of oxindole of

for (A), not greater than about 0.3% based on the measurement or measurements conducted in (b),
for (B), not greater than about 0.15% based on the measurement or measurements conducted in (b), and
for (C), not greater than about 0.03% based on the measurement or measurements conducted in (b); and

d) using the batch selected in (c) to synthesize said ziprasidone composition.

**2.** A method of synthesizing a ziprasidone composition that comprises an amount of des-chlaro ziprasidone of not greater than about 1000 ppm, which method comprises:

a) acylating a composition comprising 6-chloro-1,3-dihydro-2*H*-indol-2-one and an oxindole impurity with chloroacetyl chloride by Friedel-Crafts Acylation to synthesize a composition comprising 6-chloro-5-(chloroacetyl)-1,3-dihydro-2*H*-indol. 2-one:
b) treating the composition resulting from (a) to reduce of the oxo of the chloroacetyl group therein to form a composition comprising 6-chloro-5-(2-chloroethyl)-1,3-dihydro-2*H*-indol-2-one and a 5-(2-chloroethyl)-1,3-dihydro-2*H*-indol-2-one impurity;
c) isolating a sample of the composition resulting from (b);
d) measuring the quantity of 5-(2-chloroethyl)-1,3-dihydro-2*H*-indol-2-one impurity in the isolated sample from (c):
e) determining whether or not the quantity in (d) is not greater than about 0.28%; and
f) purifying by recrystallization and/or reslurry the composition resulting from (b) if the quantity measured in (d) is greater than about 0.28% until the quantity of the 5-(2-chloroethyl)-1,3-dihydro-2*H*-indol-2-one impurity is not greater than about 0.28%, and synthesizing a ziprasidone composition from the composition so purified: or,
g) if the quantity in (d) is not greater than about 0.28%, synthesizing a ziprasidone composition from the composition of (b).

**3.** A method using HPLC for measuring the quantity of 5-(2-chloroethyl)-1,3-dihydro-2*H*-indol-2-one in a composition comprising 6-chloro-5-(2-chloroethyl)-1,3-dihydro-2*H*-indol-2-one which method comprises

a) preparing sample solution from said composition comprising 6-chloro-5-(2-chloroethyl)-1,3-dihydro-2*H*-indol-2-one by dissolving a portion of said composition In an organic solvent, followed by dilution with an organic solvent of the dissolved portion so that a concentration (weight/volume), based on the weight of said portion and volume of solvent, of about 1 mg/mL is obtained;
b) running the sample solution through a HPLC column comprising a stationary phase consisting essentially of a cyano bonded phase using a mobile phase consisting essentially of (75:13-17:8-12 v/v/v/) 0.05 M $KH_2PO_4$, pH=from 5,5-6,5,:Acetonitrile:Methanol; at a column temperature of from 30° C to 40° c; with detection by UV light at 254nm UV;
c) detecting a peak appearing at from between 8 to 10 minutes on a chromatogram resulting from (b);
d) measuring the peak area (named $A_c$) of the peak detected in (c);
e) preparing a standard from a composition consisting essentially of 5-(2-chloroethyl)-1,3-dihydro-2*H*-indol-2-one by dissolving and diluting a portion of said composition in an organic solvent such that the concentration (weight/volume) of 5-(2-chloroethyl)-1,3-dihydro-2*H*-indol-2-one, based on the weight of the portion and volume of the solvent, is about equal to a selected fraction value at or above which detection of 5-(2-chloroethyl)-1,3-dihydro-2*H*-indol-2-one in the composition comprising 6-Chloro-5-(2-chloroethyl)-1,3-dihydro-2*H*-indol-2-one is desired;
f) running the standard through a stable-bond cyano HPLC column using a mobile phase consisting essentially of (75:13-17:8-12 v/v/v/) 0.05 M $KH_2PO_4$, pH=from 5.5-6.5,:Acetonitrile:Methanol; at a column temperature of from 30° C to 40° C; with detection by UV light at 254nm UV;
g) measuring the peak area (named $A_{pur1}$) of the peak on a chromatogram resulting from (f); and
h) calculating the quantity of 5-(2-chloroethyl)-1,3-dihydro-*2H*-indol-2-one in the composition comprising 6-chloro-5-(2-chloroethyl)-1,3-dihydro-*2H*-indol-2-one by

i) calculating the Response Factor for 5-(2-chloroethryl)-1,3-dihyrdo-2*H*-indol-2-one according to the following formula:

$$R_{pur1}=(A_{pur1})(DF)/(W_{pur1})(PF)$$

wherein: $A_{pur1}$ is as defined above;
$W_{pur1}$ = weight of composition in the standard;
PF = potency factor for 5-(2-chloroethyl)-1,3-dihyrdo-*2H*-indol-2-one; and
DF = dilution factor for the standard; and

(ii) calculating the %w/w of 5-(2-chloroethyl)-1,3-dihyrdo-2*H*-indol-2-one according to the following formula:

$$\%w/w=(A_c)(DF)(100)/(R_{pur1})(W_{32})$$

wherein : $A_c$ is as defined above;
$R_{pur1}$ = Response Factor calculated in (h)(i) above;
$W_{32}$ = weight of the portion of the composition used in step (a); and
DF = dilution factor for sample solution.

**4.** A method of synthesizing a ziprasidone composition that comprises an amount of des-chloro ziprasidone of not greater than about 1000 ppm, which method comprises:

a) reducing a composition comprising 5-chloro-5-(chloroacetyl)-1,3-dihydro-2*H*-indol-2-one and a 5-(chloroacetyl)-1,3-dihydro-2*H*-indol-2-one impurity by treatment with triethylsilane in the presence of a strong acid to obtain a composition comprising 6-chloro-5-(2-chloroetheyl-1,3-dihydro-2*H*-indol-2-one and a 5-(2-chloroetheyl-1,3-dihydro-2*H*-indol-2-one impurity; and
b) synthesizing a composition comprising ziprasidone from the composition resulting from (a).

**5.** A method according to claim 4, further comprising

i) isolating prior to step (b) a sample of the composition from (a), and measuring the quantity of 5-(2-chloroethyl)-1,3-dihydro-2*H*-indol-2-one impurity in said sample;
ii) determining whether or not the quantity in (i) is not greater than an amount selected from about 0.28%; and
iii) purifying by recrystallization and/or reslurry the composition from (a) If the quantity in (i) is greater than about 0.20% until the quantity of the 5-(2-chloroethyl)-1,3-dihydro-2*H*-indol-2-one impurity is not greater, than about 0.28%, and then proceeding to step (b) using the composition from (a) so purified; or
iv) if the quantity in (i) is not greater than about 0.28%, then proceeding to step (b).

**6.** A method of synthesizing a ziprasidone composition that comprises an amount of des-chloro ziprasidone of not greater than an amount selected from

A) about 1000 ppm,
B) about 500 ppm, and
C) about 100 ppm,

which method comprises:

a) purifying a composition comprising 6-chloro-1,3-dihydro-2*H*-indol-2-one and an oxindole impurity until a composition comprising an amount of said oxindole impurity of

for (A), about 0.3%
for (B), about 0.15%,
and for (C), about 0.03%

is obtained; and
b) using the composition resulting from (a) to synthesize a ziprasidone composition.

**7.** A method of synthesizing a ziprasidone composition that comprises an amount of des-chloro ziprasidone of not greater than about

A) 1000 ppm.
B) 500 ppm, or
C) 100 ppm;

which method comprises:

a) recrystallizing and/or reslurrying a composition comprising 6-chloro-5-(2-chloroethyl)-1,3-dihydro-2*H*-indol-2-one and a 5-(2-chloroethyl)-1,3-dihydro-2*H*-indol-2-one impurity until a composition comprising not greater than about

for (A), 0.3%,
for (B), 0.15%, and
for (C), 0.03%

of said 5-(2-chloroethyl)-1,3-dihydro-2*H*-indol-2-one impurity is obtained; and
b) using the composition resulting from (a) to synthesize a ziprasidone composition.

**Patentansprüche**

1. Verfahren zur Synthese einer Ziprasidonzusammensetzung, die eine Menge an Des-chlor-ziprasidon umfasst, die aus einer Menge von nicht größer als etwa

A) 1000 ppm,
B) nicht größer als etwa 500 ppm und
C) nicht größer als etwa 100 ppm ausgewählt ist,

wobei das Verfahren

a) das Gewinnen von einer oder mehreren Proben von einer oder mehreren Chargen von 6-Chlor-1,3-dihydro-2H-indol-2-on,
b) das Ermitteln der Konzentrationsmenge einer Oxindolverunreinigung in jeder der Proben von (a),
c) das Wählen einer Charge von 6-Chlor-1,3-dihydro-2H-indol-2-on, die eine Konzentrationsmenge von Oxindol

für (A) von nicht größer als etwa 0,3 % auf der Basis der Messung oder der Messungen, die in (b) durchgeführt wurden,
für (B) von nicht größer als etwa 0,15 % auf der Basis der Messung oder der Messungen, die in (b) durchgeführt wurden, und
für (C) von nicht größer als etwa 0,03 % auf der Basis der Messung oder der Messungen, die in (b) durchgeführt wurden, umfasst, und

d) die Verwendung der in (c) gewählten Charge zur Synthese der Ziprasidonzusammensetzung umfasst.

2. Verfahren zur Synthese einer Ziprasidonzusammensetzung, die eine Menge an Des-chlor-ziprasidon von nicht größer als etwa 1000 ppm umfasst, wobei das Verfahren

a) das Acylieren einer Zusammensetzung, die 6-Chlor-1,3-dihydro-2H-indol-2-on und eine Oxindolverunreinigung umfasst, mit Chloracetylchlorid durch eine Friedel-Crafts-Acylierung zur Synthese einer Zusammensetzung, die 6-Chlor-5-(chloracetyl)-1,3-dihydro-2H-indol-2-on umfasst;
b) das Behandeln der von (a) erhaltenen Zusammensetzung zur Reduktion der Oxogruppe der Chloracetylgruppe darin unter Bildung einer Zusammensetzung, die 6-Chlor-5-(2-chlorethyl)-1,3-dihydro-2H-indol-2-on und eine 5-(2-Chlorethyl)-1,3-dihydro-2H-indol-2-on-Verunreinigung umfasst;
c) das Isolieren einer Probe der von (b) erhaltenen Zusammensetzung;
d) das Ermitteln der Menge einer 5-(2-Chlorethyl)-1,3-dihydro-2H-indol-2-on-Verunreinigung in der isolierten Probe von (c);
e) das Bestimmen, ob die Menge in (d) nicht mehr als etwa 0,28 % beträgt oder nicht; und
f) das Reinigen der von (b) erhaltenen Zusammensetzung durch Umkristallisieren und/oder Wiederaufschlämmen, wenn die in (d) ermittelte Menge mehr als etwa 0,28 % beträgt, bis die Menge der 5-(2-Chlorethyl)-1,3-dihydro-2H-indol-2-on-Verunreinigung nicht mehr als etwa 0,28 % beträgt, und die Synthese einer Ziprasidonzusammensetzung aus der so gereinigten Zusammensetzung; oder
g) wenn die Menge in (d) nicht mehr als etwa 0,28 % beträgt, die Synthese einer Ziprasidonzusammensetzung aus der Zusammensetzung von (b) umfasst.

3. Verfahren unter Verwendung von HPLC zur Ermittlung der Menge an 5-(2-Chlorethyl)-1,3-dihydro-2H-indol-2-on

in einer Zusammensetzung, die 6-Chlor-5-(2-chlorethyl)-1,3-dihydro-2H-indol-2-on umfasst, wobei das Verfahren die folgenden Stufen umfasst:

a) Herstellen einer Probenlösung aus der Zusammensetzung, die 6-Chlor-5-(2-chlorethyl)-1,3-dihydro-2H-indol-2-on umfasst, durch Auflösen eines Teils der Zusammensetzung in einem organischen Lösemittel und anschließendes Verdünnen des gelösten Teils mit einem organischen Lösemittel so, dass eine Konzentration (Gewicht/Volumen), bezogen auf das Gewicht des Teils und das Volumen des Lösemittels, von etwa 1 mg/ml erhalten wird;
b) Laufenlassen der Probenlösung über eine HPLC-Säule, die eine im wesentlichen aus einer cyanomodifizierten gebundenen Phase bestehende stationäre Phase umfasst, unter Verwendung einer mobilen Phase, die im wesentlichen aus (75:13-17:8-12 V/V/V) 0,05 M $KH_2PO_4$, pH-Wert von 5,5-6,5,: Acetonitril:Methanol besteht, bei einer Säulentemperatur von 30 ˚C bis 40 ˚C mit Detektion durch UV-Licht bei 254 nm UV;
c) Detektieren eines Peaks, der bei zwischen 8 und 10 Minuten auf einem von (b) erhaltenen Chromatogramm erscheint;
d) Ermitteln der Peakfläche (mit der Bezeichnung $A_c$) des in (c) detektierten Peaks;
e) Herstellen eines Standards aus einer Zusammensetzung, die im wesentlichen aus 5-(2-Chlorethyl)-1,3-dihydro-2H-indol-2-on besteht, durch Auflösen und Verdünnen eines Teils der Zusammensetzung in einem organischen Lösemittel derart, dass die Konzentration (Gewicht/Volumen) von 5-(2-Chlorethyl)-1,3-dihydro-2H-indol-2-on, bezogen auf das Gewicht des Teils und das Volumen des Lösemittels, etwa gleich einem gewählten Fraktionswert, bei dem oder über dem die Detektion von 5-(2-Chlorethyl)-1,3-dihydro-2H-indol-2-on in der 6-Chlor-5-(2-chlorethyl)-1,3-dihydro-2H-indol-2-on umfassenden Zusammensetzung gewünscht wird, ist;
f) Laufenlassen des Standards über eine HPLC-Säule mit stabil gebundenem Cyano unter Verwendung einer mobilen Phase, die im wesentlichen aus (75:13-17:8-12 V/V/V) 0,05 M $KH_2PO_4$, pH-Wert von 5, 5-6, 5, : Acetonitril:Methanol besteht, bei einer Säulentemperatur von 30 ˚C bis 40 ˚C mit Detektion durch UV-Licht bei 254 nm UV;
g) Ermitteln der Peakfläche (mit der Bezeichnung $A_{pur1}$) des Peaks auf einem von (f) erhaltenen Chromatogramm und
h) Berechnen der Menge von 5-(2-Chlorethyl)-1,3-dihydro-2H-indol-2-on in der 6-Chlor-5-(2-chlorethyl)-1,3-dihydro-2H-indol-2-on umfassenden Zusammensetzung durch

i) Berechnen des Response Factor für 5-(2-Chlorethyl)-1,3-'dihydro-2H-indol-2-on gemäß der folgenden Formel:

$$R_{pur1} = (A_{pur1})(DF)/(W_{pur1})(PF)$$

worin: $A_{pur1}$ wie oben definiert ist,
$W_{pur1}$ = Gewicht der Zusammensetzung im Standard,
PF = Potenzfaktor für 5-(2-Chlorethyl)-1,3-dihydro-2H-indol-2-on und
DF = Verdünnungsfaktor für den Standard und
ii) Berechnen der % (Gew/Gew) von 5-(2-Chlorethyl)-1,3-dihydro-2H-indol-2-on gemäß der folgenden Formel:

$$\%(Gew/Gew) = (A_c)(DF)(100)/(R_{pur1})(W_{S2})$$

worin: $A_c$ wie oben definiert ist;
$R_{pur1}$ = Response Factor, der in obigem (h) (i) berechnet wurde;
$W_{S2}$ = Gewicht des in Stufe (a) verwendeten Teils der Zusammensetzung und
DF = Verdünnungsfaktor für die Probenlösung.

**4.** Verfahren zur Synthese einer Ziprasidonzusammensetzung, die eine Menge an Des-chlor-ziprasidon, die nicht größer als etwa 1000 ppm ist, umfasst, wobei das Verfahren

a) das Reduzieren einer Zusammensetzung, die 6-Chlor-5-(chloracetyl)-1,3-dihydro-2H-indol-2-on und eine

5-(Chloracetyl)-1,3-dihydro-2H-indol-2-on-Verunreinigung umfasst, durch eine Behandlung mit Triethylsilan in Gegenwart einer starken Säure, wobei eine Zusammensetzung erhalten wird, die 6-Chlor-5-(2-chlorethyl)-1,3-dihydro-2H-indol-2-on und eine 5-(2-Chlorethyl)-1,3-dihydro-2H-indol-2-on-Verunreinigung umfasst; und
b) die Synthese einer Zusammensetzung die Ziprasidon umfasst, aus der von (a) erhaltenen Zusammensetzung umfasst.

**5.** Verfahren nach Anspruch 4, das ferner

i) vor der Stufe (b) das Isolieren einer Probe der Zusammensetzung von (a) und das Ermitteln der Menge an 5-(2-Chlorethyl)-1,3-dihydro-2H-indol-2-on-Verunreinigung in der Probe;
ii) das Bestimmen, ob die Menge in (i) nicht größer als eine gewählte Menge von etwa 0,28 % ist oder nicht; und
iii) das Reinigen der Zusammensetzung von (a) durch Umkristallisieren und/oder Wiederaufschlämmen, wenn die Menge in (i) mehr als etwa 0,28 % beträgt, bis die Menge an der 5-(2-Chlorethyl)-1,3-dihydro-2H-indol-2-on-Verunreinigung nicht größer als etwa 0,28 % ist, und dann das Weitergehen zu Stufe (b) unter Verwendung der so gereinigten Zusammensetzung von (a); oder
iv) wenn die Menge in (i) nicht mehr als etwa 0,28 % beträgt, dann das Weitergehen zu Stufe (b) umfasst.

**6.** Verfahren zur Synthese einer Ziprasidonzusammensetzung, die eine Menge an Des-chlor-ziprasidon umfasst, die nicht größer als eine aus

A) etwa 1000 ppm,
B) etwa 500 ppm und
C) etwa 100 ppm ausgewählte Menge ist,

wobei das Verfahren

a) das Reinigen einer Zusammensetzung, die 6-Chlor-1,3-dihydro-2H-indol-2-on und eine Oxindol-Verunreinigung umfasst, bis eine Zusammensetzung erhalten wird, die eine Menge der Oxindol-Verunreinigung von

für (A) etwa 0,3 %,
für (B) etwa 0,15 % und
für (C) etwa 0,03 % umfasst; und

b) die Verwendung der von (a) erhaltenen Zusammensetzung zur Synthese einer Ziprasidonzusammensetzung umfasst.

**7.** Verfahren zur Synthese einer Ziprasidonzusammensetzung, die eine Menge an Des-chlor-ziprasidon umfasst, die nicht größer als etwa

A) 1000 ppm,
B) 500 ppm oder
C) 100 ppm ist;

wobei das Verfahren

a) das Umkristalliseren und/oder Wiederaufschlämmen einer Zusammensetzung, die 6-Chlor-5-(2-chlorethyl)-1,3-dihydro-2H-indol-2-on und eine 5-(2-Chlorethyl)-1,3-dihydro-2H-indol-2-on-Verunreinigung umfasst, bis eine Zusammensetzung erhalten wird, die nicht mehr als etwa

für (A) 0,3 %,
für (B) 0,15 % und
für (C) 0,03 % an der 5-(2-Chlorethyl)-1,3-dihydro-2H-indol-2-on-Verunreinigung umfasst; und

b) die Verwendung der von (a) erhaltenen Zusammensetzung zur Synthese einer Ziprasidonzusammensetzung umfasst.

## Revendications

1. Procédé de synthèse d'une composition de ziprasidone qui comprend une quantité de déchloroziprasidone choisie pour être non supérieure à environ

   A) 1000 ppm,
   B) non supérieure à environ 500 ppm, et
   C) non supérieure à environ 100 ppm

   lequel procédé comprend les étapes consistant à :

   a) obtenir un ou plusieurs échantillons d'un ou plusieurs lots de 6-chloro-1,3-dihydro-2H-indole-2-one ;
   b) mesurer le taux d'oxindole présent comme impureté dans chacun des échantillons de a) ;
   c) choisir un lot de 6-chloro-1,3-dihydro-2*H*-indole-2-one qui comprend un taux d'oxindole

      pour (A), non supérieur à environ 0,3 % d'après la mesure ou les mesures effectuées en (b),
      pour (B), non supérieur à environ 0,15 % d'après la mesure ou les mesures effectuées en (b), et
      pour (C), non supérieur à environ 0,03 % d'après la mesure ou les mesures effectuées en (b) ; et

   d) utiliser le lot choisi en (c) pour synthétiser ladite composition de ziprasidone.

2. Procédé de synthèse d'une composition de ziprasidone qui comprend une quantité de déchloroziprasidone non supérieure à environ 1000 ppm, lequel procédé comprend les étapes consistant à :

   a) acyler une composition comprenant de la 6-chloro-1,3-dihydro-2H-indole-2-one et de l'oxindole comme impureté avec du chlorure de chloracétyle par acylation de Friedel-Crafts pour synthétiser une composition comprenant de la 6-chloro-5-(chloracétyl)-1,3-dihydro-2*H*-indole-2-one ;
   b) traiter la composition résultant de (a) pour réduire le groupe oxo de son groupe chloracétyle pour former une composition comprenant de la 6-chloro-5-(2-chloréthyl)-1,3-dihydro-2*H*-indole-2-one et de la 5-(2-chloréthyl)-1,3-dihydro-2*H*-indole-2-one comme impureté ;
   c) isoler un échantillon de la composition résultant de (b) ;
   d) mesurer la quantité de la 5-(2-chloréthyl)-1,3-dihydro-2*H*-indole-2-one présente comme impureté dans l'échantillon isolé provenant de (c) ;
   e) déterminer si la quantité mesurée en (d) est ou non supérieure à environ 0,28 % ; et
   f) purifier par recristallisation et/ou remise en suspension la composition résultant de (b) si la quantité mesurée en (d) est supérieure à environ 0,28 %, jusqu'à ce que la quantité de la 5-(2-chloréthyl)-1,3-dihydro-2*H*-indole-2-one présente comme impureté ne soit pas supérieure à environ 0,28 %, et synthétiser une composition de ziprasidone à partir de la composition ainsi purifiée ; ou
   g) si la quantité mesurée en (d) n'est pas supérieure à environ 0,28 %, synthétiser une composition de ziprasidone à partir de la composition de (b).

3. Procédé utilisant la CLHP pour mesurer la quantité de 5-(2-chloréthyl)-1,3-dihydro-2*H*-indole-2-one dans une composition comprenant de la 6-chloro-5-(2-chloréthyl)-1,3-dihydro-2*H*-indole-2-one, lequel procédé comprend les étapes consistant à :

   a) préparer une solution d'échantillon à partir de ladite composition comprenant de la 6-chloro-5-(2-chloréthyl)-1,3-dihydro-2*H*-indole-2-one en dissolvant une portion de ladite composition dans un solvant organique, puis en diluant avec un solvant organique la portion dissoute de façon à obtenir une concentration (poids/volume), sur la base du poids de ladite portion et du volume de solvant, d'environ 1 mg/ml ;
   b) faire passer la solution d'échantillon à travers une colonne de CLHP comprenant une phase fixe constituée essentiellement d'une phase à liaison cyano en utilisant une phase mobile constituée essentiellement de $KH_2PO_4$ 0,05 M, pH = 5,5 à 6,5:acétonitrile:méthanol (75:13-17:8-12 en volume/volume/volume) ; à une température de la colonne de 30˚C à 40˚C ; avec détection par la lumière UV à 254 nm UV ;
   c) détecter un pic apparaissant à un temps de 8 à 10 minutes sur un chromatogramme résultant de (b) ;
   d) mesurer l'aire de pic (dénommée $A_c$) du pic détecté en (c) ;
   e) préparer un étalon à partir d'une composition constituée essentiellement de 5-(2-chloréthyl)-1,3-dihydro-2*H*-indole-2-one en dissolvant et diluant une portion de ladite composition dans un solvant organique de telle façon que la concentration (poids/volume) de 5-(2-chloréthyl)-1,3-dihydro-2*H*-indole-2-one, sur la base du poids

de la portion et du volume de solvant, soit à peu près égale à une valeur de fraction choisie à laquelle ou au-dessus de laquelle on souhaite la détection de 5-(2-chloréthyl)-1,3-dihydro-2*H*-indole-2-one dans la composition comprenant la 6-chloro-5-(2-chloréthyl)-1,3-dihydro-2*H*-indole-2-one ;

f) faire passer l'étalon à travers une colonne de CLHP cyano à liaison stable en utilisant une phase mobile constituée essentiellement de $KH_2PO_4$ 0,05 M, pH = 5,5 à 6,5:acétonitrile:méthanol (75:13-17:8-12 en volume/volume/volume) ; à une température de la colonne de 30˚C à 40˚C ; avec détection par la lumière UV à 254 nm UV ;

g) mesurer l'aire de pic (dénommée $A_{pur1}$) du pic sur un chromatogramme résultant de (f) ; et

h) calculer la quantité de 5-(2-chloréthyl)-1,3-dihydro-2*H*-indole-2-one dans la composition comprenant la 6-chloro-5-(2-chloréthyl)-1,3-dihydro-2*H*-indole-2-one

i) en calculant le Facteur de Réponse pour la 5-(2-chloréthyl)-1,3-dihydro-2*H*-indole-2-one selon la formule suivante :

$$R_{pur1} = (A_{pur1})(FD)/(P_{pur1})(FA)$$

où : $A_{pur1}$ est tel que défini ci-dessus ;

$P_{pur1}$ = poids de composition dans l'étalon ;
FA = facteur d'activité pour la 5-(2-chloréthyl)-1,3-dihydro-2*H*-indole-2-one ; et
FD = facteur de dilution pour l'étalon ;
et

ii) en calculant le % en poids/poids de 5-(2-chloréthyl)-1,3-dihydro-2*H*-indole-2-one selon la formule suivante :

$$\%\ en\ poids = (A_c)(FD)(100)/(R_{pur1})(P_{S2})$$

où : $A_c$ est tel que défini ci-dessus ;

$R_{pur1}$ = Facteur de Réponse calculé en (h) (i) ci-dessus ;
$P_{S2}$ = poids de la portion de la composition utilisée dans l'étape (a); et
FD = facteur de dilution pour la solution d'échantillon.

**4.** Procédé de synthèse d'une composition de ziprasidone qui comprend une quantité de déchloro-ziprasidone non supérieure à environ 1000 ppm, lequel procédé comprend les étapes consistant à :

a) réduire une composition comprenant de la 6-chloro-5-(chloracétyl)-1,3-dihydro-2*H*-indole-2-one et de la 5-(chloracétyl)-1,3-dihydro-2*H*-indole-2-one comme impureté par traitement avec du triéthylsilane en présence d'un acide fort pour obtenir une composition comprenant de la 6-chloro-5-(2-chloréthyl)-1,3-dihydro-2*H*-indole-2-one et de la 5-(2-chloréthyl)-1,3-dihydro-2*H*-indole-2-one comme impureté ; et

b) synthétiser une composition comprenant de la ziprasidone à partir de la composition résultant de (a).

**5.** Procédé selon la revendication 4, consistant de plus à :

i) isoler, avant l'étape (b), un échantillon de la composition provenant de (a), et mesurer la quantité de 5-(2-chloréthyl)-1,3-dihydro-2*H*-indole-2-one présente comme impureté dans ledit échantillon ;

ii) déterminer si la quantité mesurée en (i) est ou non supérieure à une quantité choisie d'environ 0,28 % ; et

iii) purifier par recristallisation et/ou remise en suspension la composition provenant de (a) si la quantité mesurée en (i) est supérieure à environ 0,28 %, jusqu'à ce que la quantité de la 5-(2-chloréthyl)-1,3-dihydro-2H-indole-2-one présente comme impureté ne soit pas supérieure à environ 0,28 %, puis procéder à l'étape (b) en utilisant la composition provenant de (a) ainsi purifiée ; ou

iv) si la quantité mesurée en (i) n'est pas supérieure à environ 0,28 %, alors procéder à l'étape (b).

**6.** Procédé de synthèse d'une composition de ziprasidone qui comprend une quantité de déchloroziprasidone non supérieure à une quantité choisie parmi

A) environ 1000 ppm,
B) environ 500 ppm, et
C) environ 100 ppm,

lequel procédé comprend les étapes consistant à

a) purifier une composition comprenant de la 6-chloro-1,3-dihydro-2*H*-indole-2-one et de l'oxindole comme impureté jusqu'à obtention d'une composition comprenant une quantité dudit oxindole présent comme impureté de

pour (A), environ 0,3 %
pour (B), environ 0,15 %
et pour (C), environ 0,03 % ;

et
b) utiliser la composition résultant de (a) pour synthétiser une composition de ziprasidone.

**7.** Procédé de synthèse d'une composition de ziprasidone qui comprend une quantité de déchloroziprasidone non supérieure à environ

A) 1000 ppm,
B) 500 ppm, ou
C) 100 ppm ;

lequel procédé comprend les étapes consistant à :

a) recristalliser et/ou remettre en suspension une composition comprenant de la 6-chloro-5-(2-chloréthyl)-1,3-dihydro-2*H*-indole-2-one et de la 5-(2-chloréthyl)-1,3-dihydro-2*H*-indole-2-one comme impureté jusqu'à obtention d'une composition ne contenant pas plus d'environ

pour (A), 0,3 %,
pour (B), 0,15 %, et
pour (C), 0,03 %

de ladite 5-(2-chloréthyl)-1,3-dihydro-2*H*-indole-2-one présente comme impureté ; et
b) utiliser la composition résultant de (a) pour synthétiser une composition de ziprasidone.